(19) Europäisches Patentamt
European Patent Office
Office européen des brevets

(11) Veröffentlichungsnummer: **0 134 400 B1**

(12) **EUROPÄISCHE PATENTSCHRIFT**

(45) Veröffentlichungstag der Patentschrift: **24.03.93**

(51) Int. Cl.⁵: **C07D 401/12**, A61K 31/44, A61K 31/415, C07D 235/28

(21) Anmeldenummer: **84104993.5**

(22) Anmeldetag: **03.05.84**

Die Akte enthält technische Angaben, die nach dem Eingang der Anmeldung eingereicht wurden und die nicht in dieser Patentschrift enthalten sind.

# (54) Anti-sekretorische, Fluoralkoxy-substituierte Pyridylmethylthio-(oder sulfinyl-)benzimidazole.

(30) Priorität: **03.05.83 CH 2401/83**

(43) Veröffentlichungstag der Anmeldung:
**20.03.85 Patentblatt 85/12**

(45) Bekanntmachung des Hinweises auf die Patenterteilung:
**24.03.93 Patentblatt 93/12**

(84) Benannte Vertragsstaaten:
**AT BE CH DE FR GB IT LI LU NL SE**

(56) Entgegenhaltungen:
**EP-A- 146 370**
**EP-A- 234 690**
**EP-A- 246 126**
**EP-A- 251 536**
**EP-A- 254 588**
**EP-A- 0 005 129**
**EP-A- 0 074 341**
**CH-A- 642 359**
**DE-A- 3 603 362**
**GB-A- 2 022 093**
**LU-A- 77 115**
**LU-A- 77 125**
**US-A- 4 435 406**

**Gastroenterology, 1968;55: 125-134**

**Naunyn-Schmiedeberg's Arch. Parmacol, 1982, 320: 170-174**

(73) Patentinhaber: **Byk Gulden Lomberg Chemische Fabrik GmbH**
**Byk-Gulden-Strasse 2**
**W-7750 Konstanz(DE)**

(72) Erfinder: **Rainer, Georg, Dr.**
**J.A. Feuchtmayer-Str. 7**
**W-7750 Konstanz(DE)**
Erfinder: **Riedel, Richard, Dr.**
**Salmannsweilerg. 36**
**W-7750 Konstanz(DE)**
Erfinder: **Senn-Bilfinger, Jörg, Dr.**
**Renkenweg 12**
**W-7750 Konstanz(DE)**
Erfinder: **Klemm, Kurt, Prof. Dr.**
**Im Weinberg 2**
**W-7753 Allensbach(DE)**
Erfinder: **Schaefer, Hartmann, Dr.**
**Zum Purren 27**
**W-7750 Konstanz 16(DE)**
Erfinder: **Figala, Volker, Dr.**
**Am Hochfürst 2**
**W-7753 Allensbach 4(DE)**

**EP 0 134 400 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Hakanson R, Hedenbro J, Liedberg G. Sundler F and Vallgren S,J. Physiol. 1980;305:139-149

Okabe S, Takeuchi K, Nakamura K and Takagi K, Am J Dig Dis 1975;20:138-147

Guth PH, Pausen G, Lynn d and Aures D, Gastroenterology 1976;71:750-753

Bristol JA, Gold EH and Lovey RG, J. Med. Chem. 1981;24:927-932

Cheng HC, Gleason EM, Nathan BA, Lachmann PG and Woodward JK, J.Pharm. Exp. Ther. 1981;217:121-126

Bass PA and Patterson MA, J Pharmacol, Exp. Ther. 1967;156:142-148

Shay H, Komarov SA, Fels SS, Meranze D, Gruenstein M and Siplet H, Gastroenterology 1945;5:43-61

Fromm D, Am, J. Surg. 1978;135:379-385

Okabe S, Takeuchi K, Nakamuka K and Takagi K, Jap. J. Pharmacol. 1974;24:363-371

Kasuya Y, Murata T and Okabe S, Jap. J. Pharmacol. 1978;28:297-304

Gastroenterology 1983; 85: 900-907

Okabe S, Takeuchi K, Urushidani T and Takagi K, Am.J. Dig. Dis. 1977; 22.677-684

Okabe S, Honda K, Takeuchi K and Takagi K, Am. J. Dig. Dis. 1975; 20:626-631

Okabe S, Takeuchi K, Honda K and Takagi K, Anrzneim. Forsch/Drug. Res. 1976; 26: 534-537

Takeuchi K Okabe S and Takagi K, Am. J. Dig. Dis. 1976;21:853-858

Okabe S, TakataY, Takeuchi K Naganuma T and Takagi K, Am. J. Dig. Dis. 1976; 21: 618-625

Okabe S, Takeguchi K, Urushidani T, Naganuma T and Takagi K, Jap. J Pharmacol. 1975; 25: 687-691

Urushidani T, Okabe S, Takeuchi K and Takagi K, Jap. J. Pharmacol. 1978; 28: 569-578

Okabe S, Hung C-R, Takeguchi K and Takagi K, Jap, J. Pharmacol. 1976; 26: 455-460

Tanaka H, Suzuki Y, Kojima T and Marumo H, Jap J. Pharmacol. 1976; 26: 403-410

Journal of Chromatography, 1984; 309: 347-356

Okabe S, Takeuchi K, Honda K and Takagi K, Jap. J. Pharmacol. 1974; 24: 490-492

Okabe S and Kunimi H, Jap. J Pharmacol. 1981; 31: 941-950

Guth PH, Aures D and Paulsen G, Gastroenterology 1979; 76:88-93

Kauffman GI and Grossman MI, Gastroenterology 1978; 75: 1099-1102

Murukami M, Oketani K, Fujisaki H, Wakabayashi T and Ohgo T, Arzneim Forsch/Drug Res. 1981; 31: 799-804

## Beschreibung

Anwendungsgebiet der Erfindung

Die Erfindung betrifft neue Fluoralkoxyverbindungen, Verfahren zu ihrer Herstellung, ihre Anwendung und sie enthaltende Arzneimittel. Die erfindungsgemäßen Verbindungen werden in der pharmazeutischen Industrie als Zwischenprodukte und zur Herstellung von Medikamenten verwendet.

Stand der Technik

In der europäischen Patentanmeldung 0 005 129 werden substituierte Pyridylsulfinylbenzimidazole beschrieben, die magensäuresekretionshemmende Eigenschaften besitzen. - In der europäischen Patentanmeldung 0 074 341 wird die Verwendung einer Reihe von Benzimidazolderivaten zur Magensäuresekretionshemmung beschrieben.

Es wurde nun überraschenderweise gefunden, daß die unten näher beschriebenen Fluoralkoxyverbindungen interessante und unerwartete Eigenschaften aufweisen, durch die sie sich in vorteilhafter Weise von den bekannten Verbindungen unterscheiden.

Beschreibung der Erfindung

Gegenstand der Erfindung sind neue Fluoralkoxyverbindungen der allgemeinen Formel I

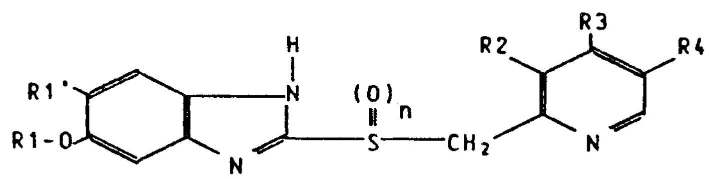

(I),

worin

- R1 einen ganz oder überwiegend durch Fluor substituierten 1-3C-Alkylrest oder einen Chlordifluormethylrest,
- R1' Wasserstoff, Halogen, Trifluormethyl, einen 1-36-Alkylrest oder einen gegebenenfalls ganz oder überwiegend durch Fluor substituierten 1-3C-Alkoxyrest,
- R2 Wasserstoff oder einen 1-3C-Alkylrest,
- R3 Wasserstoff, einen 1-3C-Alkyl- oder einen 1-3C-Alkoxyrest.
- R4 Wasserstoff oder einen 1-3C-Alkylrest und
- n die Zahlen 0 oder 1 darstellen,

sowie die Salze dieser Verbindungen.

Als ganz oder überwiegend durch Fluor substituierte 1-3C-Alkylreste seien beispielsweise der 1,1,2-Trifluorethylrest, der Perfluorpropylrest, der Perfluorethylrest und insbesondere der 1,1,2,2-Tetrafluorethylrest, der Trifluormethylrest, der 2,2,2-Trifluorethylrest und der Difluormethylrest genannt.

Halogen im Sinne der vorliegenden Erfindung ist Brom, Chlor, und insbesondere Fluor.

1-3C-Alkylreste sind der Propyl-, Isopropyl-, Ethyl- und insbesondere der Methylrest.

1-3C-Alkoxyreste enthalten neben dem Sauerstoffatom die vorstehend genannten 1-36-Alkylreste. Bevorzugt ist der Methoxyrest.

Ganz oder überwiegend durch Fluor substituierte 1-3C-Alkoxyreste enthalten neben dem Sauerstoffatom die vorstehend aufgeführten ganz oder überwiegend durch Fluor substituierten 1-3C-Alkylreste. Beispielsweise seien der 1,1,2,2-Tetrafluorethoxy-, der Trifluormethoxy-, der 2,2,2-Trifluorethoxy- und insbesondere der Difluormethoxyrest genannt.

Als Salze kommen für Verbindungen der Formel I, in denen n die Zahl 0 bedeutet (Sulfide), bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid,

Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 2-Hydroxy-3-naphthoat. 3-Hydroxy-2-naphthoat oder Mesilat.

Für Verbindungen der Formel I, in denen n die Zahl 1 bedeutet (Sulfoxide), kommen als Salze bevorzugt basische Salze in Betracht, insbesondere pharmakologisch verträgliche Salze mit in der Galenik üblicherweise verwendeten anorganischen und organischen Basen. Als Beispiele für basische Salze seien Natrium-, Kalium-, Calcium- oder Aluminiumsalze erwähnt.

Eine Ausgestaltung (Ausgestaltung a) der Erfindung sind Verbindungen der allgemeinen Formel I, worin R1 einen ganz oder überwiegend durch Fluor substituierten 1-3C-Alkylrest und R1' Wasserstoff, Halogen, Trifluormethyl oder einen ganz oder überwiegend durch Fluor substituierten 1-3C-Alkoxyrest darstellen; R2, R3, R4 und n die eingangs angegebenen Bedeutungen haben, und die Salze dieser Verbindungen.

Eine weitere Ausgestaltung (Ausgestaltung b) der Erfindung sind Verbindungen der allgemeinen Formel I, worin R1 einen ganz oder überwiegend durch Fluor substituierten 1-3C-Alkylrest darstellt und R1', R2, R3, R4 und n die eingangs angegebenen Bedeutungen haben, sowie die Salze dieser Verbindungen.

Eine weitere Ausgestaltung (Ausgestaltung c) der Erfindung sind Verbindungen der allgemeinen Formel I, worin R1' Wasserstoff, Halogen, Trifluormethyl oder einen ganz oder überwiegend durch Fluor substituierten 1-3C-Alkoxyrest darstellt und R1, R2, R3, R4 und n die eingangs angegebenen Bedeutungen haben, sowie die Salze dieser Verbindungen.

Hervorzuhebende erfindungsgemäße Verbindungen sind solche der allgemeinen Formel I, in denen R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff, Fluor, Methoxy oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind; n die Zahlen 0 oder 1 darstellt, und die Salze dieser Verbindungen.

Hervorzuhebende Verbindungen der Ausgestaltung a sind solche der allgemeinen Formel I, worin R1 einen Trifluormethylrest, einen 1,1,2,2-Tetrafluorethylrest, einen 2,2,2-Trifluorethylrest oder einen Difluormethylrest darstellt; R1' Wasserstoff, Fluor oder Difluormethoxy bedeutet; R2 Wasserstoff oder Methyl. R3 Wasserstoff oder Methoxy und R4 Wasserstoff oder Methyl bedeuten, wobei R2, R3 und R4 nicht gleichzeitig Wasserstoffatome sind; n die Zahlen 0 oder 1 darstellt, und die Salze dieser Verbindungen.

Hervorzuhebende Verbindungen der Ausgestaltung b sind solche der allgemeinen Formel I, worin R1 einen Trifluormethylrest, einen 1,1,2,2-Tetrafluorethylrest, einen 2,2,2-Trifluorethylrest oder einen Difluormethylrest darstellt; R1' Wasserstoff, Fluor, Methoxy oder Difluormethoxy bedeutet; R2 Wasserstoff oder Methyl, R3 Wasserstoff oder Methoxy und R4 Wasserstoff oder Methyl bedeuten. wobei R2, R3 und R4 nicht gleichzeitig Wasserstoffatome sind; n die Zahlen 0 oder 1 darstellt, und die Salze dieser Verbindungen.

Hervorzuhebende Verbindungen der Ausgestaltung c sind solche der allgemeinen Formel 1, worin R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff. Fluor oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind; n die Zahlen 0 oder 1 darstellt, und die Salze dieser Verbindungen.

Bevorzugte erfindungsgemäße Verbindungen sind solche der allgemeinen Formel I, in denen R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff bedeutet; R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

Bevorzugte erfindungsgemäße Verbindungen sind weiterhin solche der allgemeinen Formel I, in denen R1 einen Difluormethylrest darstellt, R1' Fluor, Methoxy oder Difluormethoxy bedeutet, R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

Bevorzugte Verbindungen der Ausgestaltung a sind solche der allgemeinen Formel I, worin R1 einen Trifluormethylrest, einen 1,1,2,2-Tetrafluorethylrest, einen 2,2,2-Trifluorethylrest oder einen Difluormethylrest darstellt; R1' Wasserstoff bedeutet; R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

Bevorzugte Verbindungen der Ausgestaltung b sind solche der allgemeinen Formel I, worin R1 einen Trifluormethylrest, einen 1,1,2,2-Tetrafluorethylrest, einen 2,2,2-Trifluorethylrest oder einen Difluormethylrest darstellt; R1' Fluor, Methoxy oder Difluormethoxy bedeutet; R2 Wasserstoff oder Methyl, R3 Methoxy und

R4 Wasserstoff oder Methyl bedeuten, n die Zahlen 0 oder 1 darstellt, und die pharmakologisch verträglichen Salze dieser Verbindungen.

Bevorzugte Verbindungen der Ausgestaltung c sind solche der allgemeinen Formel I, worin R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff bedeutet; R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

Als erfindungsgemäße Verbindungen seien beispielsweise genannt:
2-[(4-Ethoxy-3-methyl-2-pyridyl)methylthio]-5-trifluormethoxy-1H-benzimidazol,
2-[(4-Ethoxy-3-methyl-2-pyridyl)methylsulfinyl]-5-trifluormethoxy-1H-benzimidazol,
2-[(3-Methyl-2-pyridyl)methylthio]5-trifluormethoxy-1H-benzimidazol,
2-[(3-Methyl-2-pyridyl)methylsulfinyl]-5-trifluormethoxy-1H-benzimidazol.
2-[(5-Methyl-2-pyridyl)methylthio]-5-trifluormethoxy-1H-benzimidazol,
2-[(5-Methyl-2-pyridyl)methylsulfinyl]-5-trifluormethoxy-1H-benzidazol,
2-[(3,5-Dimethyl-2-pyridyl)methylthio-5-trifluormethoxy-1H-benzimidazol,
2-[(3,5-Dimethyl-2-pyridyl)methylsulfinyl]-5-trifluormethoxy-1H-benzimidazol,
2-[(4-Ethoxy-3-methyl-2-pyridyl)methylthio]-5-(1,1,2,2-tetrafluorethoxy)-1H-benzimidazol,
2-[(4-Ethoxy-3-methyl-2-pyridyl)methylsulfinyl]-5-(1,1,2,2-tetrafluorethoxy)-1H-benzimidazol,
2-[(4-Methoxy-3-methyl-2-pyridyl)methylthio]-5-pentafluorethoxy-1H-benzimidazol,
2-[(4-Methoxy-3-methyl-2-pyridyl)methylsulfinyl]-5-pentafluorethoxy-1H-benzimidazol,
2-[(4-Methoxy-5-methyl-2-pyridyl)methylthio]-5-pentafluorethoxy-1H-benzimidazol,
2-[(4-Methoxy-5-methyl-2-pyridyl)methylsulfinyl)-5-pentafluorethoxy-1H-benzimidazol,
5-Heptafluorpropoxy-2-[(4-methoxy-3-methyl-2-pyridyl)methylthio]-1H-benzimidazol,
5-Heptafluorpropoxy-2-[(4-methoxy-3-methyl-2-pyridyl)methylsulfinyl]-1H-benzimidazol,
5-Heptafluorpropoxy-2-[(4-methoxy-5-methyl-2-pyridyl)methylthio]-1H-benzimidazol,
5-Heptafluorpropoxy-2-[(4-methoxy-5-methyl-2-pyridyl)methylsulfinyl)-1H-benzimidazol,
2-[(4-Methoxy-5-methyl-2-pyridyl)methylthio]-5-(2,2,2-trifluorethoxy)-1H-benzimidazol,
2-[(4-Methoxy-5-methyl-2-pyridyl)methylsulfinyl]-5-(2,2,2-trifluorethoxy)-1H-benzimidazol,
2-[(4-Methoxy-3,5-dimethyl-2-pyridyl)methylthio]-5-(2,2,2-trifluorethoxy)-1H-benzimidazol,
2-[(4-Methoxy-3,5-dimethyl-2-pyridyl)methylsulfinyl]-5-(2,2,2-trifluorethoxy)-1H-benzimidazol,
2-[(4-Ethoxy-3-methyl-2-pyridyl)methylthio]-5-(2,2,2-trifluorethoxy)-1H-benzimidazol,
2-[(4-Ethoxy-3-methyl-2-pyridyl)methylsulfinyl]-5-(2,2,2-trifluorethoxy)-1H-benzimidazol,
2-[(3-Methyl-2-pyridyl)methylthio]-5-(2,2,2-trifluorethoxy)-1H-benzimidazol,
2-[(3-Methyl-2-pyridyl)methylsulfinyl]-5-(2,2,2-trifluorethoxy)-1H-benzimidazol,
2-[(4-Methoxy-2-pyridyl)methylthiol]-5-(1,1,2-trifluorethoxy)-1H-benzimidazol,
2-[(4-Methoxy-2-pyridyl)methylsulfinyl]-5-(1,1,2-trifluorethoxy)-1H-benzimidazol,
2-[(4-Methoxy-3-methyl-2-pyridyl]methylthio]-5-(1,1,2-trifluorethoxy)-1H-benzimidazol,
2-[(4-Methoxy-3-methyl-2-pyridyl)methylsulfinyl]-5-(1,1,2-trifluorethoxy)-1H-benzimidazol,
2-[(4-Methoxy-5-methyl-2-pyridyl)methylthio]-5-(1,1,2-trifluorethoxy)-1H-benzimidazol,
2-[(4-Methoxy-5-methyl-2-pyridyl)methylsulfinyl]-5-(1,1,2-trifluorethoxy)-1H-benzimidazol,
2-[(4-Methoxy-3,5-dimethyl-2-pyridyl)methylthio]-5-(1,1,2-trifluorethoxy)-1H-benzimidazol,
2-[(4-Methoxy-3,5-dimethyl-2-pyridyl)methylsulfinyl]-5-(1,1,2-trifluorethoxy)-1H-benzimidazol,
2-[(4-Ethoxy-3-methyl-2-pyridyl)methylthio]-5-(1,1,2-trifluorethoxy)-1H-benzimidizol,
2-[(4-Ethoxy-3-methyl-2-pyridyl)methylsulfinyl]-5-(1,1,2-trifluorethoxy)-1H-benzimidazol,
2-[(3-Methyl-2-pyridyl)methylthio]-5-(1,1,2-trifluorethoxy)-1H-benzimidazol,
2-[(3-Methyl-2-pyridyl)methylsulfinyl]-5-(1,1,2-trifluorethoxy)-1H-benzimidazol,
6-Fluor-2-[(4-methoxy-3-methyl-2-pyridyl)methylthio]-5-trifluormethoxy-1H-benzimidazol,
6-Fluor-2-[(4-methoxy-3-methyl-2-pyridyl)methylsulfinyl]-5-trifluormethoxy-1H-benzimidazol,
2-[(4-Methoxy-3-methyl-2-pyridyl)methylthio]-5-trifluormethoxy-6-trifluormethyl-1H-benzimidazol,
2-[(4-Methoxy-3-methyl-2-pyridyl)methylsulfinyl]-5-trifluormethoxy-6-trifluormethyl-1H-benzimidazol,
5,6-Bis(trifluormethoxy)-2-[(4-methoxy-5-methyl-2-pyridyl)methylthio]-1H-benzimidazol,
5,6-Bis(trifluormethoxy)-2-[(4-methoxy-5-methyl-2-pyridyl)methylsulfinyl]-1H-benzimidazol,
2-[(3-Methyl-2-pyridyl)methylthio]-5-trifluormethoxy-6-trifluormethyl-1H-benzimidazol,
2-[(3-Methyl-2-pyridyl)methylsulfinyl]-5-trifluormethoxy-6-trifluormethyl-1H-benzimidazol,
6-Fluor-2-[(4-methoxy-3-methyl-2-pyridyl)methylthio]-5-(1,1,2,2-tetrafluorethoxy)-1H-benzimidazol,
6-Fluor-2-[(4-methoxy-3-methyl-2-pyridyl]methylsulfinyl]-5-(1,1,2,2-tetrafluorethoxy)-1H-benzimidazol,
2-[(4-Methoxy-5-methyl-2-pyridyl)methylthio]-5-(1,1,2,2-tetrafluorethoxy)-6-trifluormethyl-1H-benzimidazol,
2-[(4-Methoxy-5-methyl-2-pyridyl)methylsulfinyl]-5-(1,1,2,2-tetrafluorethoxy)-6-trifluormethyl-1H-benzimidazol,
5,6-Bis(1,1,2,2-tetrafluorethoxy)-2-[(4-methoxy-3,5-dimethyl-2-pyridyl)-methylthio]-1H-benzimidazol,
5,6-Bis[1,1,2,2-tetrafluorethoxy)-2-[(4-methoxy-3,5-dimethyl-2-pyridyl)-mechylsulfinyl]-1H-benzimidazol,
2-[(3-Methyl-2-pyridyl)methylthio]-5-(1,1,2,2-tetrafluorethoxy)-6-trifluormethoxy-1H-benzimidazol,
2-[(3-Methyl-2-pyridyl)methylsulfinyl]-5-(1,1,2,2-tetrafluorethoxy)-6-trifluormethoxy-1H-benzimidazol.
2-[(4-Methoxy-2-pyridyl)methylthio]-5-pentafluorethoxy-1H-benzimidazol,
2-[(4-Methoxy-2-pyridyl)methylsulfinyl)-5-pentafluorethoxy-1H-benzimidazol,
2-[(5-Methyl-2-pyridyl)methylthiol]-5-(1,1,2,2-tetrafluorethoxy)-1H-benzimidazol,
2-[(5-Methyl-2-pyridyl)methylsulfinyl]-5-(1,1,2,2-tetrafluorethoxy)-1H-benzimidazol,
2-[(3,5-Dimethyl-2-pyridyl)methylthio]-5-(1,1,2,2-tetrafluorethoxy)-1H-benzimidazol,
2-[(3,5-Dimethyl-2-pyridyl)methylsulfinyl]-5-(1,1,2,2-tetrafluorethoxy)-1H-benzimidazol,
2-[(4-Methoxy-2-pyridyl)methylthio]-5,6-bis(trifluormethoxy)-1H-benzimidazol,
2-[(4-Methoxy-2-pyridyl)methylsulfinyl]-5,6-bis(trifluormethoxy)-1H-benzimidazol,
2-[(4-Methoxy-3-methyl-2-pyridyl)methylthio]-5,6-bis(trifluormethoxy)-1H-benzimidazol,
2-[(4-Methoxy-3-methyl-2-pyridyl)methylsulfinyl]-5,6-bis(trifluormethoxy)-1H-benzimidazol,
2-[(4-Methoxy-2-pyridyl)methylthio]-5,6-bist(1,1,2,2-tetrafluorethoxy)-1H-benzimidazol,
2-[(4-Methoxy-2-pyridyl)methylsulfinyl]-5,6-bis(1,1,2,2-tetrafluorethoxy)-1H-benzimidazol,
2-[(4-Methoxy-3-methyl-2-pyridyl)methylthio]-5,6-bis(1,1,2,2-tetrafluorethoxy)-1H-benzimidazol,
2-[(4-Methoxy-3-methyl-2-pyridyl)methylsulfinyl]-5,6-bis(1,1,2,2-tetrafluorethoxy)-1H-benzimidazol,
5-Difluormethoxy-2-[(4-methoxy-5-methyl-2-pyridyl)methylthio]-1H-benzimidazol,
5-Difluormethoxy-2-[(4-methoxy-5-methyl-2-pyridyl)methylsulfinyl]-1H-benzimidazol,
5-Difluormethoxy-2-[(4-methoxy-3,5-dimethyl-2-pyridyl)methylthio]-1H-benzimidizol,
5-Difluormethoxy-2-[(4-methoxy-3,5-dimethyl-2-pyridyl)methylsulfinyl]-1H-benzimidazol,
5-Difluormethoxy-2-[(3-methyl-2-pyridyl)methylthio]-1H-benzimidazol,
5-Difluormethoxy-2-[(3-methyl-2-pyridyl)methylsulfinyl]-1H-benzimidazol,
5-Difluormethoxy-2-[(5-methyl-2-pyridyl)methylthio]-1H-benzimidazol,
5-Difluormethoxy-2-[(5-methyl-2-pyridyl)methylsulfinyl]-1H-benzimidazol,
5-Difluormethoxy-2-[(3,5-dimethyl-2-pyridyl)methylthio]-1H-benzimidazol,
5-Difluormethoxy-2-[(3,5-dimethyl-2-pyridyl)methylsulfinyl]-1H-benzimidazol,
5-Chlordifluormethoxy-2-[(4-methoxy-3-methyl-2-pyridyl)methylthio)-1H-benzimidazol,
5-Chlordifluormethoxy-2-[(4-methoxy-3-methyl-2-pyridyl)methylsulfinyl]-1H-benzimidazol,
5-Chlordifluormethoxy-2-[(3-methyl-2-pyridyl)methylthio]-1H-benzimidazol,
5-Chlordifluormethoxy-2-[(3-methyl-2-pyridyl)methylsulfinyl]-1H-benzimidazol,
5,6-Bis(difluormethoxy)-2-[(4-methoxy-5-methyl-2-pyridyl)methylthio]-1H-benzimidazol,
5,6-Bis(difluormethoxy)-2-[(4-methoxy-5-methyl-2-pyridyl)methylsulfinyl)-1H-benzimidazol,
5,6-Bis(difluormethoxy)-2-[(4-methoxy-3,5-dimethyl-2-pyridyl)methylthio)-1H-benzimidazol.
5,6-Bis(difluormethoxy)-2-[(4-methoxy-3,5-dimethyl-2-pyridyl)methylsulfinyl]-1H-benzimidazol,
5,6-Bis(difluormethoxy)-2-[(3-methyl-2-pyridyl)methylthio]-1H-benzimidazol,
5,6-Bis(difluormethoxy)-2-[(3-methyl-2-pyridyl)methylsulfinyl]-1H-benzimidazol,
6-Difluormethoxy-6-fluor-2-[(4-methoxy-3-methyl-2-pyridyl)methylthio]-1H-benzimidazol,
5-Difluormethoxy-6-fluor-2-[(4-methoxy-3-methyl-2-pyridyl)methylsulfinyl]-1H-benzimidazol,
5-Difluormethoxy-6-methoxy-2-[(4-methoxy-5-methyl-2-pyridyl)methylthio]-1H-benzimidazol,
5-Difluormethoxy-6-methoxy-2-[(4-methoxy-5-methyl-2-pyridyl)methylsulfinyl)-1H-benzimidazol,
5-Difluormethoxy-6-methoxy-2-[(4-methoxy-3,5-dimethyl-2-pyridyl)methylthio]-1H-benzimidazol,
5-Difluormethoxy-6-methoxy-2-[(4-methoxy-3,5-dimethyl-2-pyridyl)methylsulfinyl]-1H-benzimidazol,
5-Difluormethoxy-6-methoxy-2[(3-methyl-2-pyridyl)methylthio]-1H-benzimidazol,
5-Difluormethoxy-6-methoxy-2-[(3-methyl-2-pyridyl)methylsulfinyl)-1H-benzimidazol,
5-Difluormethoxy-6-methoxy-2-[(5-methyl-2-pyridyl)methylthio]-1H-benzimidazol,
5-Difluormethoxy-6-methoxy-2-[(5-methyl-2-pyridyl)methylsulfinyl)-1H-benzimidazol,
5-Difluormethoxy-6-methoxy-2-[(3,5-dimethyl-2-pyridyl)methylthio]-1H-benzimidazol,
5-Difluormethoxy-6-methoxy-2-[(3,5-dimethyl-2-pyridyl)methylsulfinyl]-1H-benzimidazol,
5-Methoxy-2-[(4-methoxy-2-pyridyl)methylthio]-6-trifluormethoxy-1H-benzimidazol,
5-Methoxy-2-[(4-methoxy-2-pyridyl)methylsulfinyl]-6-trifluormethoxy-1H-benzimidazol.
5-Methoxy-2-[(4-methoxy-3-methyl-2-pyridyl)methylthio]-6-trifluormethoxy-1H-benzimidazol,
5-Methoxy-2-[(4-methoxy-3-methy1-2-pyridyl)methylsulfinyl]-6-trifluormethoxy-1H-benzimidazol,
5-Methoxy-2-[(4-methoxy-5-methyl-2-pyridyl)methylthio]-6-trifluormethoxy-1H-benzimidazol,
5-Methoxy-2-[(4-methoxy-5-methyl-2-pyridyl)methylsulfinyl]-6-trifluormethoxy-1H-benzimidazol,
5-Methoxy-2-[(4-methoxy-3,5-dimethyl-2-pyridyl)methylthio]-6-trifluormethoxy-1H-benzimidazol,

5-Methoxy-2-[(4-methoxy-3,5-dimethyl-2-pyridyl)methylsulfinyl]-6-trifluormethoxy-1H-benzimidazol,
5-Methoxy-2-[(3-methyl-2-pyridyl)methylthio]-6-trifluormethoxy-1H-benzimidazol,
5-Methoxy-2-[(3-methyl-2-pyridyl)methylsulfinyl]-6-trifluormethoxy-1H-benzimidazol,
5-Methoxy-2-[(5-methyl-2-pyridyl)methylthio]-6-trifluormethoxy-1H-benzimidazol,
5-Methoxy-2-[(5-methyl-2-pyridyl)methylsulfinyl]-6-trifluormethoxy-1H-benzimidazol,
5-Methoxy-2-[(4-methoxy-2-pyridyl)methylthio]-6-(1,1,2,2-tetrafluorethoxy)-1H-benzimidazol,
5-Methoxy-2-[(4-methoxy-2-pyridyl)methylsulfinyl]-6-(1,1,2,2-tetrafluorethoxy)-1H-benzimidazol,
5-Methoxy-2-[(4-methoxy-3-methyl-2-pyridyl)methylthio]-6-(1,1,2,2-tetrafluorethoxy)-1H-benzimidazol,
5-Methoxy-2-[(4-methoxy-3-methyl-2-pyridyl)methylsulfinyl]-6-(1,1,2,2-tetrafluorethoxy)-1H-benzimidazol,
5-Methoxy-2-[(4-methoxy-5-methyl-2-pyridyl)methylthio]-6-(1,1,2,2-tetrafluorethoxy)-1H-benzimidazol,
5-Methoxy-2-[(4-methoxy-5-methyl-2-pyridyl)methylsulfinyl]-6-(1,1,2,2-tetrafluorethoxy)-1H-benzimidazol,
5-Methoxy-2-[(4-methoxy-3,5-dimethyl-2-pyridyl)methylthio]-6-(1,1,2,2-tetrafluorethoxy)-1H-benzimidazol,
5-Methoxy-2-[(4-methoxy-3,5-dimethyl-2-pyridyl)methylsulfinyl]-6-(1,1,2,2-tetrafluorethoxy)-1H-benzimidazol,
5-Difluormethoxy-2-[(4-methoxy-2-pyridyl)methylthio]-6-methyl-1H-benzimidazol,
5-Difloormethoxy-2-[(4-methoxy-2-pyridyl)methylsulfinyl]-6-methyl-1H-benzimidazol,
5-Difloormethoxy-2-[(4-methoxy-3-methyl-2-pyridyl)methylthio]-6-methyl-1H-benzimidazol,
5-Difluormethoxy-2-[(4-methoxy-3-methyl-2-pyridyl)methylsulfinyl]-6-methyl-1H-benzimidazol,
5-Difluormethoxy-1-[(4-methoxy-2-pyridyl)methylthio]-6-(1,1,2,2-tetrafluorethoxy)-1H-benzimidazol,
5-Difluormethoxy-1-[(4-methoxy-2-pyridyl)methylsulfinyl]-6-(1,1,2,2-tetrafluorethoxy)-1H-benzimidazol,
5-Difluormethoxy-1-[(4-methoxy-3-methyl-2-pyridyl)methylthio]-6-(1,1,2,2-tetrafluorethoxy)-1H-benzimidazol,
5-Difluormethoxy-1-[(4-methoxy-3-methyl-2-pyridyl)methylsulfinyl]-6-(1,1,2,2-tetrafluorethoxy)-1H-benzimidazol,
und ihre Salze.

Bedingt durch die Tautomerie im Imidazolring ist die 5-Substitution im Benzimidazol mit der 6-Substitution identisch.

Ein weiterer Gegenstand der Erfindung ist ein Verfahren zur Herstellung der Fluoralkoxyverbindungen der Formel I, worin R1, R1', R2, R3, R4 und n die oben angegebene Bedeutung haben, und ihrer Salze.

Das Verfahren ist dadurch gekennzeichnet. daß man

a) Mercaptobenzimidazole der Formel II mit Picolinderivaten III,

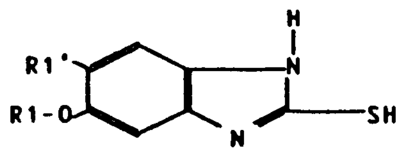 (II), 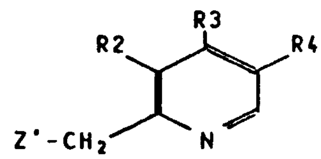 (III),

oder

b) Benzimidazole der Formel IV mit Mercaptopicolinen V,

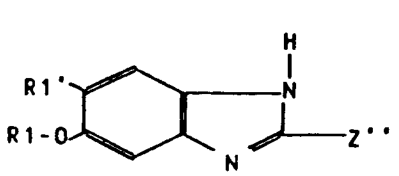 (IV), 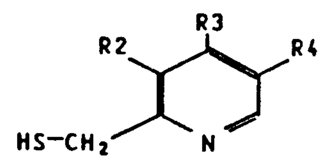 (V),

oder

c) o-Phenylendiamine der Formel VI mit Ameisensäurederivaten VII

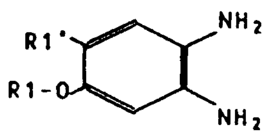 (VI), 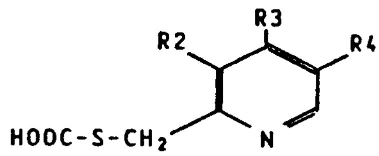 (VII),

umsetzt und gegebenenfalls anschließend die nach a), b) oder c) erhaltenen 2-Benzimidazolyl-2-pyridylmethyl-sulfide der Formel VIII

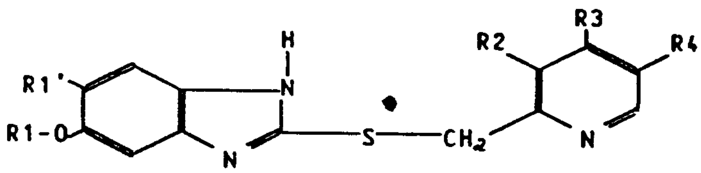 (VIII),

oxidiert und/oder in die Salze überführt,
oder daß man
d) Benzimidazole der Formel IX mit Pyridinderivaten X

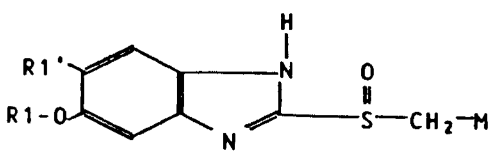 (IX), 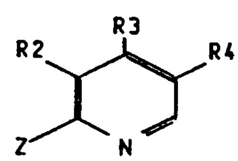 (X),

oder
e) Sulfinylderivate der Formel XI mit 2-Picolinderivaten XII

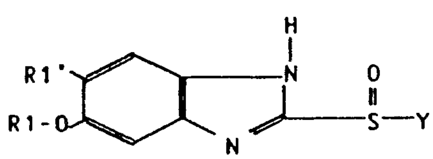 (XI), 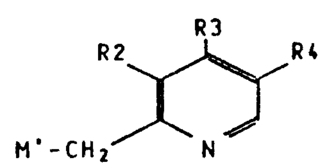 (XII),

umsetzt, und gegebenenfalls anschließend in die Salze überführt, wobei Y, Z, Z' und Z'' geeignete Abgangsgruppen darstellen. M für ein Alkalimetallatom (Li. Na oder k) steht, M' für das Äquivalent eines Metallatoms steht und R1, R1', R2, R3, R4 und n die oben angegebenen Bedeutungen haben.

Bei den vorstehend aufgeführten Umsetzungen können die Verbindungen II-XII als solche oder gegebenenfalls in Form ihrer Salze eingesetzt werden.

Die Herstellungsverfahren a), b) und c) führen zu den erfindungsgemäßen Sulfiden, die Oxidation der Verbindungen VIII und die Verfahren d) und e) liefern die erfindungsgemäßen Sulfoxide.

Welche Abgangsgruppen Y, Z, Z', bzw. Z'' geeignet sind, ist dem Fachmann aufgrund seines Fachwissens geläufig. Eine geeignete Abgangsgruppe Y ist beispielsweise eine Gruppe, die zusammen mit der Sulfinylgruppe, an die sie gebunden ist, ein reaktives Sulfinsäurederivat bildet. Als geeignete Abgangsgruppe Y seien beispielsweise Alkoxy-, Dialkylamino- oder Alkylmercaptogruppen genannt. Als geeignete Abgangsgruppen Z, Z' bzw. Z'' seien beispielsweise Halogenatome, insbesondere Chloratome, oder durch Veresterung (z.B. mit p-Toluolsulfonsäure) aktivierte Hydroxylgruppen genannt. Das Äquivalent eines Metallatoms M' ist beispielsweise ein Alkalimetall (Li, Na oder K), oder ein Erdalkalimetallatom (z.B. Mg), das durch ein Halogenatom (z.B. Br, Grignard-Reagenz) substituiert ist, oder irgendein anderes, gegebenenfalls substituiertes Metallatom, von dem bekannt ist, daß es bei Substitutionsreaktionen metallorganischer Verbindungen wie die obenerwähnten Metalle reagiert.

Die Umsetzung von II mit III erfolgt in an sich bekannter Weise in geeigneten. vorzugsweise polaren protischen oder aprotischen Lösungsmitteln (wie Methanol, Isopropanol, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei - je nach Art der Ausgangsverbindungen - gegebenenfalls zunächst die Säureadditionssalze in besonders reiner Form abgetrennt werden können. Die Reaktionstemperatur kann zwischen 60° und 150°C liegen, wobei in Gegenwart von Protonenakzeptoren Temperaturen zwischen 20° und 80°C und ohne Protonenakzeptoren zwischen 60° und 120°C - insbesondere die Siedetemperatur der verwendeten Lösungsmittel - bevorzugt sind. Die Reaktionszeiten liegen zwischen 0.5 und 12 Stunden.

Bei der Umsetzung von IV mit V, die in an sich bekannter Weise erfolgt, kommen ähnliche Reaktionsbedingungen wie bei der Umsetzung von II mit III zur Anwendung.

Die Reaktion von VI mit VII wird bevorzugt in polaren, gegebenenfalls wasserhaltigen Lösungsmitteln in Gegenwart einer starken Säure, z.B. Salzsäure, insbesondere bei der Siedetemperatur des verwendeten Lösungsmittels durchgeführt.

Die Oxidation der Sulfide VIII erfolgt in an sich bekannter Weise und - unter den Bedingungen, wie sie dem Fachmann für die Oxidation von Sulfiden zu Sulfoxiden geläufig sind [siehe hierzu z.B. J. Drabowicz und M. Mikolajczyk, Organic preparations and procedures int. 14(1-2), 45-89(1982) oder E. Block in S. Patai, The Chemistry of Functional Groups, Supplement E. Part 1. S. 539-608, John Wiley and Sons (Interscience Publication), 1980]. Als Oxidationsmittel kommen alle für die Oxidation von Sulfiden zu Sulfoxiden üblicherweise verwendeten Reagenzien in Frage, insbesondere Peroxysäuren, wie z.B. Peroxyessigsäure. Trifluorperoxyessigsäure, 3,5-Dinitroperoxybenzoesäure, Peroxymaleinsäure oder bevorzugt m-Chlorperoxybenzoesäure.

Die Reaktionstemperatur liegt (je nach Reaktivität des Oxidationsmittels und Verdünnungsgrad) zwischen -70°C und der Siedetemperatur des verwendeten Lösungsmittels, bevorzugt jedoch zwischen -30° und +20°C. Als vorteilhaft hat sich auch die Oxidation mit Halogenen bzw. mit Hypohalogeniten (z.B. mit wäßriger Natriumhypochloritlösung) erwiesen, die zweckmäßigerweise bei Temperaturen zwischen 0° und 30°C durchgeführt wird. Die Reaktion wird zweckmäßigerweise in inerten Lösungsmitteln, z. B. aromatischen oder chlorierten Kohlenwasserstoffen, wie Benzol, Toluol, Dichlormethan oder Chloroform, vorzugsweise in Estern oder Ethern, wie Essigsäureethylester, Essigsäureisopropylester oder Dioxan durchgeführt.

Die Umsetzung von IX mit X erfolgt bevorzugt in inerten Lösungsmitteln, wie sie auch für die Reaktion von Enolationen mit Alkylierungsmitteln üblicherweise verwendet werden. Beispielsweise seien aromatische Lösungsmittel wie Benzol oder Toluol genannt. Die Reaktionstemperatur liegt (je nach Art des Alkalimetallatoms M und der Abgangsgruppe Z) in der Regel zwischen 0° und 120°C, wobei die Siedetemperatur des verwendeten Lösungsmittels bevorzugt ist. Beispielsweise [wenn M Li(Lithium) und Z Cl(Chlor) darstellt und die Umsetzung in Benzol durchgeführt wird] ist die Siedetemperatur von Benzol (80°C) bevorzugt.

Die Verbindungen XI werden mit den Verbindungen XII in an sich bekannter Weise umgesetzt, wie sie dem Fachmann für die Reaktion metallorganischer Verbindungen geläufig ist.

Je nach Art der Ausgangsverbindungen, die gegebenenfalls auch in Form ihrer Salze eingesetzt werden können, und in Abhängigkeit von den Reaktionsbedingungen werden die erfindungsgemäßen Verbindungen zunächst entweder als solche oder in Form ihrer Salze gewonnen.

Im übrigen erhält man die Salze durch Auflösen der freien Verbindungen in einem geeigneten Lösungsmittel, z.B. in einem chlorierten Kohlenwasserstoff, wie Methylenchlorid oder Chloroform, einem niedermolekularen aliphatischen Alkohol (Ethanol, Isopropoanol), einem Ether (Diisopropylether), einem Keton (Aceton) oder Wasser, das die gewünschte Säure bzw. Base enthält, oder dem die gewünschte Säure bzw. Base - gegebenenfalls in der genau berechneten stöchiometrischen Menge - anschließend zugegeben wird.

Die Salze werden durch Filtrieren, Umfällen, Ausfällen oder durch Verdampfen des Lösungsmittels gewonnen.

Erhaltene Salze können durch Alkalisieren bzw. Ansäuern, z.B. mit wäßrigem Natriumhydrogencarbonat bzw. mit verdünnter Salzsäure, in die freien Verbindungen umgewandelt werden, welche wiederum in die Salze übergeführt werden können. Auf diese Weise lassen sich die Verbindungen reinigen, oder es lassen sich pharmakologisch nicht verträgliche Salze in pharmakologisch verträgliche Salze umwandeln.

Die erfindungsgemäßen Sulfoxide sind optisch aktive Verbindungen. Die Erfindung umfaßt daher sowohl die Enantiomeren als auch ihre Mischungen und Racemate. Die Enantiomeren können in an sich bekannter Weise (beispielsweise durch Herstellung und Trennung entsprechender diastereoisomerer Verbindungen) separiert werden. Die Enantiomeren können aber auch durch asymmetrische Synthese, beispielsweise

durch Reaktion optisch aktiver reiner Verbindungen XI oder diastereoisomer reiner Verbindungen XI mit Verbindungen XII hergestellt werden [siehe hierzu K.K. Andersen, Tetrahedron Lett., 93 (1962)].

Die erfindungsgemäßen Verbindungen werden bevorzugt durch Umsetzung von II mit III und gegebenenfalls anschließende Oxidation des entstandenen Sulfids VIII synthetisiert.

Die Verbindungen der Formel II sind neu und ebenfalls Gegenstand der Erfindung. Die Verbindungen III-VII sowie IX-XII sind entweder bekannt, oder sie können nach bekannten Vorschriften analog hergestellt werden. Die Verbindungen II erhält man beispielsweise durch Umsetzen von Verbindungen VI mit Kohlendisulfid in Gegenwart von Alkalihydroxiden oder mit Alkali-0-ethyldithiocarbonaten. Die Verbindungen III können gemäß O.E.Schulz u. S.Fedders, Arch. Pharm. (Weinheim)310, 128-136 (1977) hergestellt werden.

Die Verbindungen VI können nach der im folgenden Reaktionsschema A angegebenen allgemeinen Herstellungsmethode synthetisiert werden:

Reaktionsschema A:

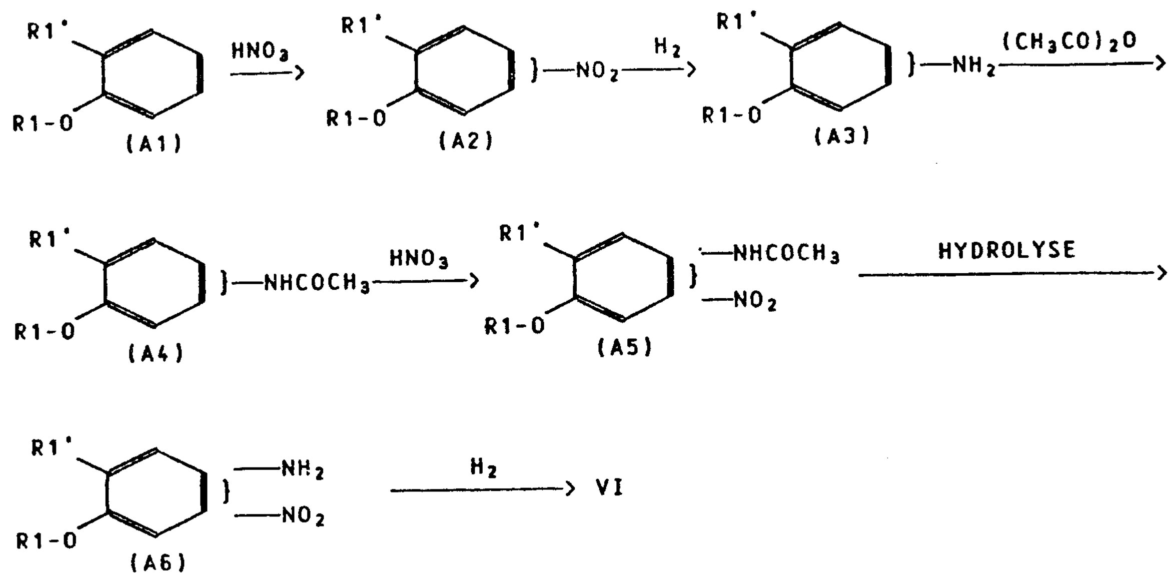


Die Ausgangsverbindungen A1-A3 können nach bekannten Methoden oder analog zu diesen [z.B. J.Org.Chem. 44, 2907-2910 (1979); J,Org.Chem. 29, 1-11 (1964); DE-OS 20 29 556; J. Fluorine Chem. 18, 281-91 (1981); Synthesis 1980, 727-8] hergestellt werden, wobei bei ungleichen Substituenten R1' und R1-0- auch Isomerengemische entstehen können.

Die Verbindungen IX werden beispielsweise aus den Verbindungen II durch Methylierung, Oxydation und anschließende Deprotonierung - z.B. mit Alkalimetallhydriden oder - alkoholaten oder üblichen metallorganischen Verbindungen - erhalten. Die Verbindungen X werden in Anlehnung an Z. Talik, Roczniki Chem 35, 475 (1961) hergestellt.

Die folgenden Beispiele erläutern die Erfindung näher ohne sie einzuschränken. Die in den Beispielen namentlich aufgeführten Verbindungen der allgemeinen Formel I sowie die Salze dieser Verbindungen sind bevorzugter Gegenstand der Erfindung. Schmp. bedeutet Schmelzpunkt, für Stunde(n) wird die Abkürzung h und für Minuten die Abkürzung Min. verwendet. Unter "Ether" wird Diethylether verstanden.

## Beispiele

1. 2-[(4-Methoxy-2-pyridyl)methylsulfinyl]-5-trifluormethoxy-1H-benzimidizol

Zu einer Lösung von 2,4 g 2-[(4-Methoxy-2-pyridyl)methylthio]-5-trifluormethoxy-1H-benzimidazol in 30 ml Dichlormethan tropft man bei -30 ° C unter gutem Rühren in 20 Min. eine Lösung von 1,45 g 85%iger m-Chlorperoxybenzoesäure in 20 ml Dichlormethan. Man rührt noch 1 h bei -30 ° C, läßt die Temperatur allmählich auf -10 ° C steigen, setzt 1 ml Triethylamin zu, wäscht die Lösung bei 0-10 ° C mit 1m Kaliumhydrogencarbonatlösung und dann mit Wasser und trocknet mit Magnesiumsulfat. Man destilliert das Lösungsmittel im Vakuum bei maximal 30 ° C ab und läßt den Rückstand aus Ether kristallisieren. Man

erhält 2.2 g (86%) der Titelverbindung. Schmp. 150-152 °C (Zersetzung).

Analog erhält man

2-[(4-Methoxy-3-methyl-2-pyridyl)methylsulfinyl]-5-trifluormethoxy-1H-benzimidazol vom Schmp. 165-166 °C (Zersetzung) aus Methanol in 65% Ausbeute,
2-[(4-Methoxy-5-methyl-2-pyridyl)methylsulfinyl]-5-trifluormethoxy-1H-benzimidazol vom Schmp. 170-172 °C (Zersetzung) aus Essigsäureethylester in 91% Ausbeute,
2-[(4-Methoxy-3,5-dimethyl-2-pyridyl)methylsulfinyl]-5-trifluormethoxy-1H-benzimidazol vom Schmp. 160-162 °C (Zersetzung) aus Essigsäureethylester in 75% Ausbeute,
2-[(4-Methoxy-2-pyridyl)methylsulfinyl]-5-(1,1,2,2-tetrafluorethoxy)-1H-benzimidazol vom Schmp. 131-132 °C (Zersetzung) aus Diisopropylether in 93% Ausbeute,
2-[(4-Methoxy-3-methyl-2-pyridyl)methylsulfinyl]-5-(1,1,2,2-tetrafluorethoxy)-1H-benzimidazol vom Schmp. 135-136 °C (Zersetzung) aus Essigsäureethylester/Petrolether (50 °C/70 °C) in 77% Ausbeute,
2-[(4-Methoxy-5-methyl-2-pyridyl)methylsulfinyl]-5-(1,1,2,2-tetrafluorethoxy)-1H-benzimidazol vom Schmp. 163-164 °C (Zersetzung) aus Diisopropylether in 93% Ausbeute,
2-[(3-Methyl-2-pyridyl)methylsulfinyl]-5-(1,1,2,2-tetrafluorethoxy)-1H-benzimidazol vom Schmp. 110-111 °C (Zersetzung) aus Diisopropylether in 61% Ausbeute,
2-[(4-Methoxy-3,5-dimethyl-2-pyridyl)methylsulfinyl]-5-(1,1,2,2-tetrafluorethoxy)-1H-benzimidazol von Schmp. 132-133 °C (Zersetzung) aus Ether in 59% Ausbeute,
2-[(4-Methoxy-2-pyridyl)methylsulfinyl]-5-(2,2,2-trifluorethoxy)-1H-benzimidazol vom Schmp. 95-97 °C aus Ether in 69% Ausbeute,
2-[(4-Methoxy-3-methyl-2-pyridyl)methylsulf1nyl]-5-(2,2,2-trifluorethoxy)-1H-benzimidazol vom Schmp. 172-173 °C (Zersetzung) aus Acetonitril in 81% Ausbeute,
5-Chlordifluormethoxy-2-[(4-methoxy-2-pyridyl)methylsulfinyl]-1H-benzimidazol vom Schmp. 154-156 °C (Zersetzung) aus Essigsäureethylester in 64% Ausbeute,
5-Difluormethoxy-2-[(4-methoxy-2-pyridyl)methylsulfinyl]-1H-benzimidazol vom Schmp. 159-161 °C (Zersetzung) aus Essigsäureethylester in 74% Ausbeute,
5-Difluormethoxy-2-[(4-methoxy-3-methyl-2-pyridyl)methylsulfinyl]-1H-benzimidazol vom Schmp. 166-168 °C (Zersetzung) aus Essigsäureethylester in 77% Ausbeute,
5,6-Bis(difluormethoxy)-2-[(4-methoxy-2-pyridyl)methylsulfinyl]-1H-benzimidazol vom Schmp. 173-175 °C (Zersetzung) aus Essigsäureethylester in 60% Ausbeute,
5,6-Bis(difluormethoxy)-2-[(4-methoxy-3-methyl-2-pyridyl)methylsulfinyl]-1H-benzimidazol vom Schmp. 184-185 °C (Zersetzung) aus Essigsäureethylester in 50% Ausbeute,
5-Difluormethoxy-6-fluor-2-[(4-methoxy-2-pyridyl)methylsulfinyl]-1H-benzimidazol vom Schmp. 160-162 °C (Zersetzung) aus Essigsäureethylester in 54% Ausbeute,
5-Difluormethoxy-6-methoxy-2-[(4-methoxy-2-pyridyl)methylsulfinyl]-1H-benzimidazol vom Schmp. 180-181 °C (Zersetsung) aus Essigsäureethylester in 88% Ausbeute und
5-Difluormethoxy-6-methoxy-2-[(4-methoxy-3-methyl-2-pyridyl)methylsulfinyl]-1H-benzimidazol vom Schmp. 94-96 °C (Zersetzung) aus Essigsäureethylester in 89% Ausbeute,
durch Umsetzung von
2-[(4-Methoxy-3-methyl-2-pyridyl)methylthio]-5-trifluormethoxy-1H-benzimidazol,
2-[(4-Methoxy-5-methyl-2-pyridyl)methylthio]-5-trifluormethoxy-1H-benzimidazol,
2-[(4-Methoxy-3,5-dimethyl-2-pyridyl)methylthio]-5-trifluormethoxy-1H-benzimidazol,
2[(4-Methoxy-2-pyridyl)methylthio]-5-(1,1,2,2-tetrafluorethoxy)-1H-benzimidazol,
2-[(4-Methoxy-3-methyl-2-pyridyl)methylthio]-5-(1,1,2,2-tetrafluorethoxy)-1H-benzimidazol,
2-[(4-Methoxy-5-methyl-2-pyridyl)methylthio]-5-[1,1,2,2-tetrafluorethoxy)-1H-benzimidazol,
2-[(3-Methyl-2-pyridyl)methylthio]-5-(1,1,2,2-tetrafluorethoxy)-1H-benzimidazol,
2-[(4-Methoxy-3,5-dimethyl-2-pyridyl)methylthio]-5-(1,1,2,2-tetrafluorethoxy)-1H-benzimidazol,
2-[(4-Methoxy-2-pyridyl)methylthio]-5-(2,2,2-trifluorethoxy)-1H-benzimidazol,
2-[(4-Methoxy-3-methyl-2-pyridyl)methylthio]-5-(2,2,2-trifluorethoxy)-1H-benzimidazol,
5-Chlordifluormethoxy-2-[(4-methoxy-2-pyridyl)methylthio]-1H-benzimidazol,
5-Difluormethoxy-2-[(4-Methoxy-2-pyridyl)methylthio]-1H-benzimidazol,
5-Difluormethoxy-2-[(4-methoxy-3-methyl-2-pyridyl)methylthio]-1H-benzimidazol,
5,6-Bis(difluormethoxy)-2-[(4-methoxy-2-pyridyl)methylthio]-1H-benzimidazol,
5,6-Bis(difluormethoxy)-2-[(4-methoxy-3-methyl-2-pyridyl)methylthio]-1H-benzimidazol,
5-Difluormethoxy-6-fluor-2-[(4-methoxy-2-pyridyl)methylthio]-1H-benzimidazol,
5-Difluormethoxy-6-methoxy-2-[(4-methoxy-2-pyridyl)methylthio]-1H-benzimidazol bzw.
5-Difluormethoxy-6-methoxy-2-[(4-methoxy-3-methyl-2-pyridyl)methylthio ]-1H-benzimidazol,
mit m-Chlorperoxybenzoesäure.

2. 2-[(4-Methoxy-2-pyridyl)methylsulfinyl]-5-(1,1,2,2-tetrafluorethoxy)-1H-benzimidazol.

Zu einer Lösung von 1.5 g 2-[(4-Methoxy-2-pyridyl)methylthio]-5-(1,1,2,2-tetrafluorethoxy)-1H-benzimidazol in 30 ml Essigsäureethylester tropft man in 20 Min. bei 0°C eine Mischung aus 8.5 ml handelsüblicher Natriumhypochloritlösung (ca. 15% aktives Chlor) und 6 ml 10%iger Natronlauge, rührt noch 20 Min. bei dieser Temperatur und setzt dann 0,6 ml 10%ige Natriumthiosulfatlösung zu. Man versetzt mit 1,5 g Ammoniumsulfat, trennt die organische Phase ab, wäscht mit gesättigter Natriumchloridlösung, trocknet mit Magnesiumsulfat und engt auf ein kleines Volumen ein. Durch Fällung mit Diisopropylether erhält man 1.3 g (85%) der Titelverbindung vom Schmp. 131-132°C (Zersetzung).

3. 2-[(4-Methoxy-2-pyridyl)methylthio]-5-trifluormethoxy-1H-benzimidazol

4,0 g 2-Merkapto-5-trifluormethoxy-1H-benzimidazol und 3.5 g 2-Chlormethyl-4-methoxypyridin-hydrochlorid werden in 100 ml Isopropanol 4,5 h unter Stickstoff zum Sieden erhitzt. Man kühlt im Eisbad und erhält 7,0 g (96%) des Dihydrochlorids der Titelverbindung vom Schmp. 164-165°C (Zersetzung). Man löst das Salz in Wasser, klärt mit Aktivkohle und setzt die Base mit Kaliumhydrogencarbonatlösung frei. Man extrahiert mit Dichlormethan, trocknet die organische Lösung mit Magnesiumsulfat und engt sie im Vakuum ein. Der Rückstand wird aus Cyclohexan kristallisiert. Man erhält 5.2 g (86%) der Titelverbindung vom Schmp. 134-135°C.

Analog erhält man
2-[(4-Methoxy-3-methyl-2-pyridyl)methylthio]-5-trifluormethoxy-1H-benzimidazol vom Schmp. 180-181°C (aus Cyclohexan) bzw.
2-[(4-Methoxy-5-methyl-2-pyridyl)methylthio]-5-trifluormethoxy-1H-benzimidazol vom Schmp. 148-149°C (aus Wasser)
durch Umsetzung von 2-Merkapto-5-trifluormethoxy-1H-benzimidazol mit 2-Chlormethyl-4-methoxy-3-methylpyridinhydrochlorid bzw. 2-Chlormethyl-4-methoxy-5-methylpyridinhydrochlorid.

Analog erhält man
2-[(4-Methoxy-2-pyridyl)methylthio]-5-(1,1,2,2-tetrafluorethoxy)-1H-benzimidazol vom Schmp. 130-131°C (aus Isopropanol),
2-[(4-Methoxy-3-methyl-2-pyridyl)methylthio]-5-(1,1,2,2-tetrafluorethoxy)-1H-benzimidazol vom Schmp. 110-111°C (aus Isopropanol),
2-[(4-Methoxy-5-methyl-2-pyridyl)methylthio]-5-(1,1,2,2-tetrafluorethoxy)-1H-benzimidazol vom Schmp. 135-136°C (aus Isopropanol), bzw. 2-[(3-Methyl-2-pyridyl)methylthio]-5-(1,1,2,2-tetrafluorethoxy)-1H-benzimidazol vom Schmp. 129-130°C (aus Isopropanol)
durch Umsetzung von 2-Merkapto-5-[1,1,2,2-tetrafluorethoxy)-1H-benzimidazol mit den Hydrochloriden von 2-Chlormethyl-4-methoxypyridin, 2-Chlormethyl-4-methoxy-3-methylpyridin, 2-Chlormethyl-4-methoxy-5-methylpyridin bzw. 2-Chlormethyl-3-methylpyridin in Isopropanol.

Analog erhält man
2-[(4-Methoxy-2-pyridyl)methylthio]-5-(2,2,2-trifluorethoxy)-1H-benzimidazol vom Schmp. 133-135°C (aus Toluol),
2-[(4-Methoxy-3-methyl-2-pyridyl)methylthio]-5-(2,2,2-trifluorethoxy)-1H-benzimidazol vom Schmp. 178-179°C (aus Toluol),
5-Chlordifluormethoxy-2-[(4-methoxy-2-pyridyl)methylthio]-1H-benzimidazol vom Schmp. 135-137°C (aus Toluol),
5-Chlordifluormethoxy-2-[(4-methoxy-3-methyl-2-pyridyl)methylthio]-1H-benzimidazol vom Schmp. 171-173°C (aus Toluol),
5-Difluormethoxy-2-[(4-methoxy-2-pyridyl)methylthio]-1H-benzimidazol vom Schmp. 115-117°C (aus Toluol),
5-Difluormethoxy-2-[(4-methoxy-3-methyl-2-pyridyl)methylthio]-1H-benzimidazol vom Schmp. 167-169°C (aus Toluol),
5,6-Bis(difluormethoxy)-2-[(4-methoxy-2-pyridyl)methylthio]-1H-benzimidazol vom Schmp. 132-134°C (aus Toluol),
5,6-Bis(difluormethoxy)-2-[(4-methoxy-3-methyl-2-pyridyl)methylthio]-1H-benzimidazol vom Schmp. 163-165°C (aus Toluol),
5-Difluormethoxy-6-fluor-2-[(4-methoxy-2-pyridyl)methylthio]-1H-benzimidazol vom Schmp. 140-142°C (aus Toluol),
5-Difluormethoxy-6-methoxy-2-[(4-methoxy-2-pyridyl)methylthio]-1HX-benzimidazol vom Schmp` 124-125°C (aus Toluol) und

5-Difluormethoxy-6-methoxy-2-[(4-methoxy-3-methyl-2-pyridyl)methylthio]-1H-benzimidazol vom Schmp. 190-191 °C (aus Toluol),
durch Umsetzung von
2-Merkapto-5-(2,2,2-trifluorethoxy)-1H-benzimidazol,
5-Chlordifluormethoxy-2-merkapto-1H-benzimidazol,
5-Difluormethoxy-2-merkapto-1H-benzimidazol,
5,6-Bis(difluormethoxy)-2-merkapto-1H-benzimidazol,
5-Difluormethoxy-6-fluor-2-merkapto-1H-benzimidazol und
5-Difluormethoxy-2-merkapto-6-methoxy-1H-benzimidazol mit 2-Chlormethyl-4-methoxypyridin bzw. 2-Chlormethyl-4-methoxy-3-methylpyridin.

4. <u>2-[(4-Methoxy-3,5-dimethyl-2-pyridyl)methylthio]-5-trifluormethoxy-1H-benzimidazol</u>

Zu einer Mischung von 2,34 g 2-Merkapto-5-trifluormethoxy-1H-benzimidazol und 2,2 g 2-Chlormethyl-4-methoxy-3,5-dimethylpyridin-hydrochlorid in 50 ml Ethanol tropft man bei Raumtemperatur 5 ml 4 m Natronlauge und rührt über Nacht bei Raumtemperatur. Man destilliert das Lösungsmittel im Vakuum ab, versetzt mit Wasser, extrahiert mit Essigsäureethylester, trocknet die Lösung und fällt mit 4 m Chlorwasserstoff in Ether 4,3 g (95%) des Dihydrochlorids der Titelverbindung. Man löst das Salz in Wasser, fällt die Base mit verdünnter Sodalösung bei pH 8 und kristallisiert aus Diisopropylether um. Man erhält 3.0 g (78%) der Titelverbindung vom Schmp. 152-154 °C.

Analog erhält man
2-[(4-Methoxy-3,5-dimethyl-2-pyridyl)methylthio]-5-(1,1,2,2-tetrafluorethoxy)-1H-benzimidazol (Schmp. 120-122 °C) durch Umsetzung von 2-Merkapto-5-(1,1,2,2-tetrafluorethoxy)-1H-benzimidazol mit 2-Chlormethyl-4-methoxy-3,5-dimethylpyridin-hydrochlorid.

5. <u>2-Merkaoto-5-(1,1,2,2-tetrafluorethoxy)-1H-benzimidazol</u>

a) 55 g 1-Nitro-4-(1,1,2,2-tetrafluorethoxylbenzol werden in 300 ml Ethanol an 0.5 g 10%iger Palladiumkohle in einer Umlaufhydrierungsapparatur unter Atmosphärendruck 1 h bei 20-45 °C hydriert, der Katalysator abfiltriert und die Lösung bei 40 °C im Vakuum eingeengt. Man verdünnt das 4-(1,1,2,2-tetrafluorethoxy)anilin mit 100 ml Eisessig und tropft 23 ml Essigsäureanhydrid bei Raumtemperatur zu, versetzt nach 30 Min. mit 2 ml Wasser, engt nach kurzer Zeit die Lösung bei 50 °C im Vakuum ein und versetzt mit 500 ml Eiswasser. Man erhält 56 g (97%) N-[4-(1,1,2,2-tetrafluorethoxy)phenyl]-acetamid vom Schmp. 121-122 °C.
b) Man löst 55 g der vorstehenden Verbindung in 380 ml Dichlormethan, tropft 55 ml 100%ige Salpetersäure in 10 Min. bei Raumtemperatur zu und rührt noch 6 h. Die organische Lösung wird dann mit wässriger Natriumcarbonatlösung und Wasser gewaschen, mit Magnesiumsulfat getrocknet und eingeengt. Man erhält 65 g (100%) N-[2-Nitro-4-(1,1,2,2-tetrafluorethoxy)-phenyl]-acetamid vom Schmp. 80-81 °C (aus Cyclohexan).
c) Man löst 63 g vorstehender Verbindung in 450 ml Methanol, tropft bei Raumtemperatur 106 ml 6 m Natronlauge zu, kühlt im Eisbad und fällt durch Zutropfen von 900 ml Wasser 53 g (98%) 2-Nitro-4-(1,1,2,2-tetrafluorethoxy)-anilin (Schmp. 85-86 °C).
d) 33 g vorstehender Verbindung werden in ca. 600 ml Isopropanol an 1 g 10%iger Palladiumkohle in einer Umlaufhydrierungsapparatur drucklos bei Raumtemperatur hydriert. Man saugt den Katalysatör ab und fällt mit 4 m Chlorwasserstoff in Ether 34 g (89%) 4-(1,1,2,2-Tetrafluorethoxy)-1,2-phenylendiamin-dihydrochlorid vom Schmp. 275-276 °C (Zersetzung).
e) 33 g vorstehender Verbindung werden mit 330 ml Etha 60 ml Wasser, 8,9 g Natriumhydroxid und 23 g Kalium-O-ethyldithiocarbonat (umkristallisiert aus Isopropanol) versetzt und 15 h unter Rückfluß zum Sieden erhitzt. Man versetzt mit 1,2 l Eiswasser, stellt mit Natronlauge auf pH 13-14, klärt mit Aktivkohle und fällt mit verdünnter Salzsäure bis pH 3,5. Man erhält 27 g (91%) der Titelverbindung vom Schmp. 316-319 °C (aus Isopropanol).

6. <u>2-Merkapto-5-trifluormethoxy-1H-benzimidazol</u>

Analog Beispiel 5e) erhält man durch Umsetzen von 4-Trifluormethoxy-1,2-phenylendiamin-dihydrochlorid (vgl. C.A. <u>55</u>, 23408d, 1961) mit Kalium-O-ethyldithiocarbonat und Natronlauge in Ethanol in 75% Ausbeute die Titelverbindung vom Schmp. 305-307 °C (Zersetzung, aus Toluol).

7. 2-Merkapto-5-(2,2,2-trifluorethoxy)-1H-benzimidazol

a) 50 g 1-(2,2,2-Trifluorethoxy)-4-nitrobenzol (Synthesis 1980, Seite 721) werden analog Beispiel 5a hydriert und acetyliert. Man erhält 50 g (95%) N-[4-(2,2,2-Trifluorethoxy)phenyl]-acetamid (Schmp. 140-141 ° C).
b) Man rührt 42 g voranstehender Verbindung mit 9,7 ml 100% Salpetersäure in 290 ml Eisessig 18 h bei Raumtemperatur und fällt mit Wasser. Man erhält 47 g (94%) N-[2-Nitro-4-(2,2,2-trifluorethoxy)-phenyl]-acetamid
c) Man hydrolysiert 47 g voranstehender Verbindung analog Beispiel 5c und erhält 38,7 g [97%) 2-Nitro-4-(2,2,2-trifluorethoxy)-anilin (Schmp. 84-85 ° C).
d) Man hydriert 37 g voranstehender Verbindung analog Beispiel 5d und erhält 41 g (94%) 4-(2,2,2-Trifluorethoxy)-1,2-phenylendiamin-dihydrochlorid vom Schmp. 230-233 ° C (Zersetzung).
e) Analog Beispiel 5e erhält man aus 36 g voranstehender Verbindung 30 g (94%) der Titelverbindung (Schmp. 288-290 ° C).

8. 5-Chlordifluormethoxy-2-merkapto-1H-benzimidazol

a) 10.0 g N-[4-(Chlordifluormethoxy)phenyl]-acetamid (Schmp. 101-103 ° C, aus 4-Chlordifluormethoxyanilin und Essigsäureanhydrid) und 12,3 ml 100% Salpetersäure werden in 80 ml Dichlormethan 4 h bei 20 ° C gerührt. Man neutralisiert mit wäßriger Kaliumhydrogencarbonatlösung, engt die organische Schicht ein und erhält 11,4 g (96%) N-(4-Chlordifluormethoxy-2-nitrophenyl)-acetamid (Schmp. 89-91 ° C).
b) Man tropft bei 5 ° C zu 10.5 g voranstehender Verbindung in 200 ml Methanol 8.6 ml einer 30%igen Lösung von Natriummethylat in Methanol, rührt 2 h ohne Kühlung, versetzt mit Eiswasser, stellt auf pH 8 und erhält 8,7 g (97%) 4-Chlordifluormethoxy-2-nitroanilin (Schmp. 40-42 ° C).
c) Man hydriert 8,5 g voranstehender Verbindung an 0.8 g 10%iger Palladiumkohle drucklos in 200 ml Methanol, versetzt mit konzentrierter Salzsäure, filtriert, engt ein und verrührt mit Diisopropylether. Man erhält 8.5 g (97%) 4-Chlordifluormethoxy-1,2-phenylendiamin-dihydrochlorid.
d) Aus 8.5 g voranstehender Verbindung werden analog Beispiel 5e 6,3 g (72%) der Titelverbindung vom Schmp. 268-270 ° C (Zersetzung) erhalten.

9. 5-Difluormethoxy-2-merkapto-1H-benzimidazol

a) 11,8 g N-(4-Difluormethoxyphenyl)-acetamid [L.M.Jagupol'skii et al., J.General Chemistry (USSR) 39, 190 (1969)] werden in 200 ml Dichlormethan mit 12.1 ml 100%iger Salzsäure 1,5 h bei Raumtemperatur gertührt. Analog Beispiel 5b erhält man 13,3 g (92%) N-[(4-Difluormethoxy-2-nitro)phenyl]-acetamid (Schmp. 71-73 ° C).
b) Analog Beispiel 8b erhält man daraus in 96%iger Ausbeute 4-Difluormethoxy-2-nitroanilin (Schmp. 68-70 ° C).
c) Analog Beispiel 8c erhält man in 94% Ausbeute 4-Difluormethoxy-1,2-phenylendiamin-dihydrochlorid.
d) Analog Beispiel 5e erhält man in 78% Ausbeute die Titelverbindung vom Schmp. 250-252 ° C (aus Isopropanol).

10. 5,6-Bis(difluormethoxy)-2-merkapto-1H-benzimidazol

a) In eine Lösung von 100 g Brenzkatechin, 220 g Natriumhydroxid und 60 g Natriumdithionit in 500 ml Wasser und 400 ml Dioxan leitet man bei 50-55 ° C 275 g Chlordifluormethan analog L.N. Sedova et al., Zh. Org. Khim. 6, 568 (1970) ein. Man erhält nach Destillation bei 61-62 ° C/1,0-1,1kPa eine Mischung von 1,2-Bis(difluormethoxy)benzol und 2-Difluormethoxyphenol, die durch Chromatographie an Kieselgel mittels Cyclohexan/Essigsäureethylester (4:1) getrennt werden.
b) Eine Lösung von 15 g 1,2-Bis(difluormethoxy)benzol und 15 ml 100%iger Salpetersäure in 150 ml Dichlormethan wird 7 h bei Raumtemperatur gerührt. Man neutralisiert mit Kaliumhydrogencarbonatlösung, trennt die organische Schicht ab und chromatographiert an Kieselgel mittels Cyclohexan/Essigsäureethylester (4:1). Man erhält 1,2-Bis(difluormethoxy)-4-nitrobenzol. Dieses hydriert und acetyliert man analog Beispiel 5a zu N-[3,4-Bis(difluormethoxy)phenyl]acetamid (Schmp. 81-83 ° C). Analog Beispiel 5 erhält man ferner N-[4,5-Bis(difluormethoxy)-2-nitrophenyl]acetamid (Schmp. 65-67 ° C), N-[4,5-Bis(difluormethoxy)-2-nitro]anilin (Schmp. 107-109 ° C), 4,5-Bis(difluormethoxy)-1,2-phenylendiamin-dihydrochlorid und die Titelverbindung vom Schmp. 285-287 ° C (Zersetzung; aus Isopropanol).

11. 5-Difluormethoxy-2-merkapto-6-methoxy-1H-benzimidazol

a) In eine Lösung von 55,5 g Guajacol und 130 g Natriumhydroxid in 300 ml Wasser und 300 ml Dioxan werden bei 60 ° C ca. 58 g Chlordifluormethan eingeleitet. Man filtriert die Mischung bei 10 ° C, trennt die organische Schicht ab, trocknet mit wasserfreiem Kaliumcarbonat und destilliert. Man erhält 56 g (73%) 1-Difluormethoxy-2-methoxybenzol vom Siedepunkt 75-76 ° C/0,9kPa.

b) Zu einer Lösung von 47 g voranstehender Verbindung in 230 ml Dichlormethan wird bei 0-5 ° C eine Lösung von 33,8 ml 100%iger Salpetersäure in 90 ml Dichlormethan getropft, nach 30 Min. mit 250 ml Eiswasser versetzt und mit Kaliumhydrogencarbonat neutralisiert. Die getrocknete organische Phase wird im Vakuum eingeengt und der Rückstand aus Cyclohexan umkristallisiert. Man erhält 53 g (90%) 1-Difluormethoxy-2-methoxy-5-nitrobenzol [Schmp. 48-49 ° C). Dieses wird analog Beispiel 5a hydriert und acetyliert. Man erhält in 90% Ausbeute N-(3-Difluormethoxy-4-methoxyphenyl)acetamid (Schmp. 129-130 ° C).

c) 46 g voranstehender Verbindung werden mit 33 ml 100%iger Salpetersäure in Dichlormethan analog voranstehender Vorschrift nitriert. Man erhält in 99% Ausbeute N-(5-Difluormethoxy-4-methoxy-2-nitrophenyl)acetamid (Schmp. 116-117 ° C).

d) 54 g voranstehender Verbindung werden in 810 ml Methanol 1 h mit 44,8 ml 30%iger methanolischer Natriummethylatlösung bei Raumtemperatur gerührt. Man engt im Vakuum ein, versetzt mit Eiswasser und Eisessig bis pH 8 und erhält in 99% Ausbeute 5-Difluormethoxy-4-methoxy-2-nitroanilin (Schmp. 144-145 ° C).

e) 25 g voranstehender Verbindung werden in 300 ml Methanol an 1,25 g 10%iger Palladiumkohle entsprechend Beispiel 5d hydriert. Man erhält 26 g (88%) 3-Difluormethoxy-4-methoxy-1,2-phenylendiamindihydrochlorid vom Schmp. 218-220 ° C (Zersetzung).

f) 25 g voranstehender Verbindung werden mit 19 g Kalium-0-ethyldithiocarbonat entsprechend Beispiel 5e umgesetzt. Man erhält 20 g (89%) der Titelverbindung vom Schmp. 280-282 ° C (Zersetzung; aus Isopropanol).

12. 5-Difluormethoxy-6-fluor-2-merkapto-1H-benzimidazol

a) Analog Beispiel 11a erhält man aus 2-Fluorphenol und Chlordifluormethan 1-Difluormethoxy-2-fluorbenzol (Sdp. 76 ° C/10 kPa; $n_D^{20}$ = 1,4340)

b) Zu 30 g der voranstehenden Verbindung in 300 ml Dichlormethan tropft man bei -10 ° C 38,4 ml 100%ige Salpetersäure, rührt 1 h bei -10 ° C und 2,5 h bei 0 ° C. Man versetzt mit Eiswasser, stellt neutral und chromatographiert über Kieselgel mit Essigester/Cyclohexan (4:1). Man erhält 34 g eines Öles, das ca. 90% 1-Difluormethoxy-2-fluor-4-nitrobenzol und 10% 1-Difluormethoxy-2-fluor-5-nitrobenzol (NMR-Spektrum) enthält.

c) 30 g voranstehender Mischung wird analog Beispiel 5a hydriert und acetyliert. Nach Umkristallisieren aus Toluol erhält man 21 g (65%) N-(4-Difluormethoxy-3-fluorphenyl)acetamid vom Schmp. 112-113 ° C.

d) Zu 20 g voranstehender Verbindung in 200 ml Dichlormethan werden bei 20 ° C 22,5 ml 100%ige Salpetersäure in 30 Min. zugetropft und 15 h bei Raumtemperatur nachgerührt. Analog Beispiel 11c erhält man in 89% Ausbeute N-(4-Difluormethoxy-5-fluor-2-nitrophenyl)acetamid vom Schmp. 72-74 ° C (aus Cyclohexan). Durch mehrstündiges Rühren mit 1 m Salzsäure in Methanol bei 60 ° C erhält min in 95% Ausbeute 4-Difluormethoxy-5-fluor-2-nitroanilinvom Schmp. 95-97,5 ° C und analog Beispiel 11e in 85% Ausbeute 4-Difluormethoxy-5-fluor-1,2-phenylendiamin-dihydrochlorid. Zersetzung ab 210 ° C.

e) 15 g voranstehender Verbindung werden mit 11,8 g Kalium-0-ethyldithiocarbonat entsprechend Beispiel 5e umgesetzt. Man erhält 11,1 g (84%) der Titelverbindung vom Schmp. 275-276 ° C (Zersetzung aus Isopropanol).

13. 2-[(4-Methoxy-2-pyridyl)methylsulfinyl]-5-(1,1,2,2-tetrafluorethoxy)-1H-benzimidazol-Natriumsalz

2.017 g 2-[(4-methoxy-2-pyridyl)methylsulfinyl]-5-(1,1,2,2-tetrafluorethoxy)-1H-benzimidazol werden mit 50 ml 0,1 m Natronlauge und 50 ml Aceton versetzt. Die Lösung wird am Rotationsverdampfer bei 60 ° C eingedampft und der Rückstand mit Ether zur Kristallisation gebracht. Anschließend trocknet man bei 60 ° C im Vakuum über Calciumchlorid und erhält die Titelverbindung als Hydrat.

Analog erhält man

2-[(4-Methoxy-2-pyridyl)methylsulfinyl]-5-trifluormethoxy-1H-benzimidazol-Natriumsalz,

2-[(4-Methoxy-3-methyl-2-pyridyl)methylsulfinyl]-5-trifluormethoxy-1H-benzimidazol-Natriumsalz und
5-Difluormethoxy-6-methoxy-2-[(4-methoxy-3-methyl-2-pyridyl)methylsulfinyl]-1H-benzimidazol-Natriumsalz.

14. 2-Chlormethyl-4-methoxypyridin-hydrochlorid

Zu einer auf -10 ° C gekühlten Lösung von 10 g (0,072 Mol) 2-Hydroxymethyl-4-methogypyridin in 30 ml trockenem Chloroform werden 15 ml Thionylchlorid innerhalb von 15 Minuten zugetropft. Man läßt die Lösung auf Raumtemperatur kommen und rührt noch eineinhalb Stunden. Nach Abziehen des Lösungsmittels und des überschüssigen Thionylchlorids erhält man farblose Kristalle, die aus Isopropanol umkristallisiert werden [12.1 g (87%), Schmp. 149-150 ° C• Zersetzung].

In analoger Weise erhält man durch Umsetzung von
2-Hydroxymethyl-4-methoxy-3-methylpyridin,
2-Hydroxymethyl-4-methoxy-3,5-dimethylpyridin,
2-Hydroxymethyl-4-methoxy-5-methylpyridin bzw.
2-Hydroxymethyl-3-methylpyridin mit Thionylchlorid
2-Chlormethyl-4-methoxy-3-methylpyridin-hydrochlorid (Schmp. 157-158 ° C, Zersetzung, aus Isopropanol/Ether),
2-Chlormethyl-4-methoxy-3,5-dimethylpyridin-hydrochlorid [Schmp. 135-136 ° C (Zersetzung) aus Isopropanol/Ether],
2-Chlormethyl-4-methoxy-5-methylpyridin-hydrochlorid (Schmp. 147 ° C, Zersetzung) bzw.
2-Chlormethyl-3-methylpyridin-hydrochlorid (Schmp. 163-165 ° C).

Die Hydroxymethyl-pyridine (siehe auch Beispiel 15) werden in Anlehnung an bzw. nach Vorschrift von O.E. Schulz und S. Fedders, Arch. Pharm. (Weinheim) 310, 128 (1977) erhalten. Die entsprechend benötigten Vorprodukte werden gemäß H.C. Brown, S. Johnson und H. Podall, J.Am.Chem.Soc. 76, 5556 (1954) hergestellt.

15. 2-Hydroxymethyl-4-methoxy-3,5-dimethylpyridin-hydrochlorid

18 g 2,3,5-Trimethylpyridin [F. Bohlmann, A. Englisch. J. Politt, H. Sander und W. Weise, Chem.Ber. 88, 1831 (1955)] und 17 ml 30%iges Wasserstoffperoxid werden in 80 ml Eisessig 2,5 h bei 100 ° C erwärmt. Danach setzt man weitere 10 ml 30%iges Wasserstoffperoxid zu und hält die Temperatur weitere 8 h. Das Gemisch wird anschließend im Wasserstrahlvakuum auf das halbe Volumen eingeengt und einem Peroxidtext unterzogen. Bei Peroxidfreiheit wird alles Lösungsmittel im Vakuum abgezogen und der Rückstand im Hochvakuum destilliert. Bei 95-98 ° C und 0,01 Torr (1,33 Pa) gehen 19,2 g (95%) 2,3,5-Trimethylpyridin-N-oxid über.

5.0 g hiervon werden bei Raumtemperatur in einem Gemisch aus 7 ml rauchender Salpetersäure und 7 ml konzentrierter Schwefelsäure gelöst und 18 Stunden bei 40 ° C Badtemperatur erwärmt. Danach gießt man auf Eiswasser und stellt mit starker Natronlauge unter Kühlung alkalisch. Die Extraktion des Gemisches mit Essigsäureethylester ergibt nach Entfernung des Lösungsmittels im Vakuum rohes 2,3,5-Trimethyl-4-nitropryridin-N-oxid, das ohne weitere Reiningung in 20 ml trockenem Methanol gelöst wird. Zu dieser Lösung gibt man 4.7 ml käufliches 30%iges Natriumethoxid in Methanol und erwärmt 12 h auf 50 ° C. Danach wird das Lösungsmittel abgezogen; der Rückstand wird in wenig Wasser aufgenommen und mit Essigsäureethylester extrahiert. Nach Abziehen des Lösungsmittels wird das als Öl zurückbleibende rohe 4-Methoxy-2,3,5-trimethylpyridin-N-oxid ohne weitere Reinigung in 20 ml 100 ° C heißes Essigsäureanhydrid gegossen und 1 Stunde bei dieser Temperatur erwärmt. Danach wird im Vakuum eingeengt und ohne weitere Reinigung in 20 ml 10%iger wässriger Salzsäure aufgenommen und 2,5 h bei 50 ° C gerührt. Im Vakuum wird auf das halbe Volumen eingeengt, mit Kaliumcarbonat alkalisch gestellt und mit Essigsäureethylester extrahiert. Die vereinigten Extrakte werden über Natriumsulfat getrocknet; das Lösungsmittel wird im Vakuum abgezogen. Der ölige Rückstand wird in 50 ml Ethylmethylketon gelöst und mit etherischer Salzsäure bis zur quantitativen Ausfällung versetzt. Der Niederschlag wird aus Dioxan mit wenig Isopropanol umkristallisiert. Man erhält 3,1 g der Titelverbindung vom Schmp. 126 ° C. Nach Chromatographie der freien Base an einer Kieselgelsäule findet man für die freie Base Schmp. 49-51 ° C und für das Hydrochlorid nach Wiederfällung in Chlorwasserstoff/Ether Schmp. 133,5 ° C (Zersetzung).

In ähnlicher Weise wird 2-Hydroxymethyl-4-methoxy-5-methylpyridin (Schmp. 102-104 ° C) erhalten.

Gewerbliche Anwendbarkeit

Die Fluoralkoxyverbindungen der allgemeinen Formel I und ihre Salze besitzen wertvolle pharmakologische Eigenschaften, die sie gewerblich verwertbar machen. Sie hemmen deutlich die Magensäuresekretion von Warmblütern und weisen darüberhinaus eine ausgezeichnete Magen- und Darmschutzwirkung bei Warmblütern auf. Diese Magen- und Darmschutzwirkung wird bereits bei der Verabreichung von Dosen beobachtet, die unterhalb der säuresekretionshemmenden Dosen liegen. Darüberhinaus zeichnen sich die erfindungsgemäßen Verbindungen durch das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. bestimmte Antiphlogistika und Antirheumatika), Chemikalien (z.B. Ethanol), Magensäure oder Streßsituationen verursacht werden können.

Ein weiterer Vorteil der erfindungsgemäßen Verbindungen ist ihre vergleichsweise große chemische Stabililtät.

In ihren ausgezeichneten Eigenschaften erweisen sich die erfindungsgemäßen Verbindungen überraschenderweise den aus dem Stand der Technik bekannten Verbindungen deutlich überlegen. Aufgrund dieser Eigenschaften sind die Fluoralkoxyverbindungen und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Ein weiterer Gegenstand der Erfindung sind daher die erfindungsgemäßen Verbindungen zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden. Ein weiterer Gegenstand der Erfindung sind Arzneimittel, die ein oder mehrere Fluoralkoxyverbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen ( = Wirkstoffe) entweder als solche, oder vorzugsweise in Kombination mit geeigneten pharmazeutischen Hilfsstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0.1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquillizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Die Formulierung der Wirkstoffe kann z.B. auf folgende Weise erfolgen:

a) Tabletten mit 40 mg Wirkstoff

20 kg 2-[(4-Methoxy-3-methyl-2-pyridyl)methylthio]-5-trifluormethoxy-1H-benzimidazol, 40 kg Milchzucker, 26 kg Maisstärke und 3 kg Polyvinylpyrrolidon werden mit ca. 20 Liter Wasser befeuchtet und durch ein Sieb mit 1,25 mm Maschenweite granuliert. Das Granulat wird im Wirbelschichttrockner bis zu einer

relativen Feuchte von 50-60% getrocknet und dann mit 8 kg Natriumcarboxymethylcellulose, 2 kg Talkum und 1 kg Magnesiumstearat versetzt. Das fertige Granulat wird zu Tabletten a 200 mg und 8 mm Durchmesser gepreßt.

b) Kapseln mit einem Wirkstoffgehalt von 30 mg

300 g 2-[(4-Methoxy-3-methyl-2-pyridyl)methylsulfinyl]-5-trifluormethoxy-1H-benzimidazol, 695 g mikrokristalline Cellulose und 5 g amorphe Kieselsäure werden feingepulvert, gut vermischt und in Hartgelatinekapseln Größe 4 abgefüllt.

c) Kapseln mit einem Wirkstoffgehalt von 10 mg

100 g 2-[(4-Methoxy-3-methyl-2-pyridyl)methylsulfinyl]-5-(1,1,2,2-tetrafluorethoxy)-1H-benzimidazol, 895 g mikrokristalline Cellulose und 5 g amorphe Kieselsäure werden feingepulvert, gut vermischt und in Hartgelatinekapseln Größe 4 abgefüllt.

d) Ampullen enthaltend 10 mg Wirkstoff

Man löst 3,16 g 2-[(4-Methoxy-2-pyridyl)methylsulfinyl]-5-trifluormethoxy-1H-benzimidazol-Natriumsalz in einer Lösung von 165.5 g Mannit und 0.5 g Natriumcarbonat in 1300 ml destilliertem Wasser, füllt mit destilliertem Wasser auf 1500 ml auf und sterilfiltriert. Von dieser Lösung dosiert man je 5 ml in 15 ml Vials ein und lyophilisiert. Das Lyophilisat kann mit 10 ml Wasser rekonstituiert werden.

## Pharmakologie

Die ausgezeichnete Magenschutzwirkung und die magensekretionshemmende Wirkung der erfindungsgemäßen Fluoralkoxyverbindungen im Vergleich zu den Verbindungen des Standes der Technik läßt sich tierexperimentell am Modell Shay-Ratte nachweisen. Die untersuchten Verbindungen des Standes der Technik sind wie folgt mit den Buchstaben A-D, die erfindungsgemäßen Verbindungen sind wie folgt mit Nummern versehen worden:

| lfd. Nr. | Name der Verbindung |
|---|---|
| A | 5-methoxy-2-[(4-methoxy-3,5-dimethyl-2-pyridyl-methyl)thio]-1H-benzimidazol (EP 0 074 341) |
| B | 5-methoxy-2-[(4-methoxy-3,5-dimethyl-2-pyridyl-methyl)sulfinyl]-1H-benzimidazol (EP 0 005 129) (INN: Omeprazol) |
| C | 5-methoxy-2-[(4-methoxy-5-methyl-2-pyridylmethyl)-thio]-1H-benzimidazol (EP 0 074 341) |
| D | 5-methoxy-2-[(4-methoxy-5-methyl-2-pyridylmethyl)-sulfinyl]-1H-benzimidazol (EP 0 005 129) |
| 1 | 2-[(4-Methoxy-2-pyridyl)methylsulfinyl]-5-trifluor-methoxy-1H-benzimidazol |
| 2 | 2-[(4-Methoxy-3-methyl-2-pyridyl)methylthio]-5-tri-fluormethoxy-1H-benzimidazol |
| 3 | 2-[(4-Methoxy-3-methyl-2-pyridyl)methylsulfinyl]-5-trifluormethoxy-1H-benzimidazol |
| 4 | 2-[(4-Methoxy-5-methyl-2-pyridyl)methylsulfinyl]-5-trifluormethoxy-1H-benzimidazol |
| 5 | 2-[(4-Methoxy-2-pyridyl)methylsulfinyl]-5-(1,1,2,2-tetrafluorethoxy)-1H-benzimidazol |
| 6 | 2-[(4-Methoxy-3-methyl-2-pyridyl)methylsulfinyl]-5-(1,1,2,2-tetrafluorethoxy)-1H-benzimidazol |
| 7 | 2-[(4-Methoxy-3-methyl-2-pyridyl)methylthio]-5-(1,1,2,2-tetrafluorethoxy)-1H-benzimidazol |

| lfd. Nr. | Name der Verbindung |
|---|---|
| 8 | 2-[(4-Methoxy-3,5-dimethyl-2-pyridyl)methylsulfinyl]-5-trifluormethoxy-1H-benzimidazol |
| 9 | 2-[(4-Methoxy-3,5-dimethyl-2-pyridyl)methylthio]-5-trifluormethoxy-1H-benzimidazol |
| 10 | 2-[(4-Methoxy-3-methyl-2-pyridyl)methylsulfinyl]-5-(2,2,2-trifluorethoxy)-1H-benzimidazol |
| 11 | 2-[(4-Methoxy-3-methyl-2-pyridyl)methylthio]-5-(2,2,2-trifluorethoxy)-1H-benzimidazol |
| 12 | 5-Difluormethoxy-2-[(4-methoxy-3-methyl-2-pyridyl)-methylsulfinyl]-1H-benzimidazol |
| 13 | 5,6-Bis(difluormethoxy)-2-[(4-methoxy-2-pyridyl)methyl-sulfinyl]-1H-benzimidazol |
| 14 | 5,6-Bis(difluormethoxy)-2-[(4-methoxy-3-methyl-2-pyridyl)-methylsulfinyl]-1H-benzimidazol |
| 15 | 5,6-Bis(difluormethoxy)-2-[(4-methoxy-3-methyl-2-pyridyl)-methylthio]-1H-benzimidazol |
| 16 | 5-Difluormethoxy-6-methoxy-2-[(4-methoxy-3-methyl-2-pyridyl)methylsulfinyl]-1H-benzimidazol |
| 17 | 5-Difluormethoxy-6-methoxy-2-[(4-methoxy-3-methyl-2-pyridyl)methylthio]-1H-benzimidazol |
| 18 | 5-Chlordifluormethoxy-2-[(4-methoxy-2-pyridyl)methyl-sulfinyl]-1H-benzimidazol |

Der Einfluß der untersuchten Verbindungen auf die durch Pylorusligatur (4h; sog. Shay-Ratte) und orale Gabe von 100 mg/kg Acetylsalicylsäure ausgelöste Magenläsionsbildung sowie die Magensekretion (HCl) während 4 h bei der Ratte ist in der folgenden Tabelle dargestellt.

Magenschutzwirkung und Magensekretionshemmung

| Lfd. Nr. | n [Anzahl d.Tiere] | Magenschutzwirkung (Ratte) Hemmung d. Läsionsindexes, ED50+) [mg/kg, p.o.] | Magensekretion (Ratte) % Hemmung der HCl-Sekretion ++) | ED25+) [mg/kg, p.o.] | ED50+) [mg/kg, p.o.] |
|---|---|---|---|---|---|
| A | 88 | 9,0 | 20 | 12,0 | 25,0 |
| B | 112 | 2,2 | 29 | 1,9 | 4,6 |
| C | 24 | -30,0 | | | >60,0 |
| D | 40 | -5,0 | 10 | 8,0 | 18,0 |
| 1 | 79 | 0,6 | 32 | 0,5 | 1,1 |
| 2 | 16 | 0,3 | 20 | 0,35 | 0,5 |
| 3 | 55 | 0,3 | 32 | <0,3 | 0,4 |
| 4 | 74 | 0,9 | 32 | 0,6 | 1,6 |
| 5 | 70 | 0,7 | 33 | 0,5 | 1,2 |
| 6 | 55 | 0,5 | 35 | 0,3 | 0,7 |
| 7 | 24 | 0,5 | 40 | 0,35 | 0,7 |
| 8 | 24 | 0,4 | 20 | 1,0 | 1,5 |
| 9 | 24 | 1,0 | 10 | 1,4 | 2,0 |
| 10 | 24 | 0,5 | 25 | 0,5 | 0,7 |
| 11 | 24 | 0,5 | 25 | 0,5 | 0,7 |
| 12 | 16 | -0,3 | -20 | -0,3 | -0,6 |
| 13 | 16 | -1,2 | -30 | -1,1 | -1,7 |
| 14 | 32 | 0,2 | 20 | -0,3 | 0,6 |
| 15 | 32 | 0,6 | 30 | 0,5 | 1,0 |
| 16 | 32 | 0,2 | | | -0,8 |
| 17 | 32 | -0,3 | -25 | -0,3 | -0,8 |
| 18 | 16 | 1,3 | 35 | <1,0 | 1,8 |

+) ED25 bzw. ED50 =Dosis, die den Läsionsindex bzw. die HCl-Sekretion (4 h) des Rattenmagens bei der behandelten Gruppe gegenüber der Kontrollgruppe um 25 bis 50% mindert.

++) nach Gabe der antiulcerösen ED50

Die Prüfung der antiulcerogenen Wirkung erfolgte nach der Methode der sogenannten Shay-Ratte:
Die Ulcusprovokation erfolgt bei 24 Stunden nüchtern gehaltenen Ratten (weiblich, 180-200 g. 4 Tiere je Kafig auf hohem Gitterrost) durch Pylorusligatur (unter Diethylethernarkose) und orale Applikation von 100 mg/10 ml/kg Acetylsalicylsäure Die zu prüfenden Substanzen werden oral (10 ml/kg) eine Stunde vor der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens.

Der Magen wird längs eröffnet und auf einer Korkplatte fixiert, nachdem zuvor die Menge des sezernierten Magensaftes (Volumen) und später sein HCl-Gehalt (Titration mit Natronlauge) bestimmt wurde; mit einem Stereomikroskop werden bei 10-facher Vergrößerung Anzahl und Größe (= Durchmesser) vorhandener Ulcera ermittelt. Das Produkt aus Schweregrad (gemäß nachfolgender Punkteskala) und Anzahl der Ulcera dient als individueller Läsionsindex.

Punkteskala:

| | | |
|---|---|---|
| keine Ulcera | | 0 |
| Ulcusdurchmesser | 0,1 - 1,4 mm<br>1,5 - 2,4 mm<br>2,5 - 3,4 mm<br>3,5 - 4,4 mm<br>4,5 - 5,4 mm<br>> 5,5 mm | 1<br>2<br>3<br>4<br>5<br>6 |

Als Maß für den antiulcerogenen Effekt dient die Minderung des mittleren Läsionsindex jeder behandelten Gruppe gegenüber dem dar Kontrollgruppe (= 100%). Die ED25 bzw. ED50 bezeichnen diejenigen Dosen, die den mittleren Läsionsindex bzw. die HCl-Sekretion gegenüber der Kontrolle um 25% bzw. 50% mindern.

Toxizität

Die LD50 aller geprüften Verbindungen liegt oberhalb von 1000 mg/kg [p.o.] bei der Maus.

**Patentansprüche**
**Patentansprüche für folgende Vertragsstaaten : BE, CH, DE, FR, GB, IT, LT, LU, NL, SE**

**1.** Fluoralkoxyverbindungen der allgemeinen Formel I

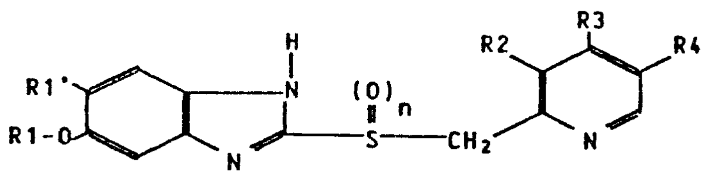

(I),

worin

- R1 einen ganz oder überwiegend durch Fluor substituierten 1-3C-Alkylrest oder einen Chlordifluormethylrest,
- R1' Wasserstoff, Halogen, Trifluormethyl, einen 1-3C-Alkylrest, oder einen gegebenenfalls ganz oder überwiegend durch Fluor substituierten 1-3C-Alkoxyrest,
- R2 Wasserstoff oder einen 1-3C-Alkylrest,
- R3 Wasserstoff, einen 1-3C-Alkyl- oder einen 1-3C-Alkoxyrest,
- R4 Wasserstoff oder einen 1-3C-Alkylrest und
- n die Zahlen 0 oder 1 darstellen,

sowie die Salze dieser Verbindungen.

**2.** Verbindungen der allgemeinen Formel I nach Anspruch 1, worin R1 einen ganz oder überwiegend durch Fluor substituierten 1-3C-Alkylrest und R1' Wasserstoff, Halogen, Trifluormethyl oder einen ganz oder überwiegend durch Fluor substituierten 1-3C-Alkoxyrest darstellen; R2, R3, R4 und n die in Anspruch 1 angegebenen Bedeutungen haben, und die Salze dieser Verbindungen.

**3.** Verbindungen der allgemeinen Formel I nach Anspruch 1, worin R1 einen ganz oder überwiegend durch Fluor substituierten 1-3C-Alkylrest darstellt und R1', R2, R3, R4 und n die in Anspruch 1 angegebenen Bedeutungen haben, sowie die Salze dieser Verbindungen.

4. Verbindungen der allgemeinen Formel I nach Anspruch 1, worin R1' Wasserstoff, Halogen, Trifluormethyl oder einen ganz oder überwiegend durch Fluor substituierten 1-3C-Alkoxyrest darstellt und R1, R2, R3, R4 und n die in Anspruch 1 angegebenen Bedeutungen haben, sowie die Salze dieser Verbindungen.

5. Verbindungen der allgemeinen Formel I nach Anspruch 1, in denen R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff, Fluor, Methoxy oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind; n die Zahlen 0 oder 1 darstellt, und die Salze dieser Verbindungen.

6. Verbindungen der allgemeinen Formel I nach Anspruch 1, worin R1 einen Trifluormethylrest, einen 1,1,2,2-Tetrafluorethylrest, einen 2,2,2-Trifluorethylrest oder einen Difluormethylrest darstellt; R1' Wasserstoff, Fluor oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind; n die Zahlen 0 oder 1 darstellt, und die Salze dieser Verbindungen.

7. Verbindungen der allgemeinen Formel I nach Anspruch 1, worin R1 einen Trifluormethylrest, einen 1,1,2,2-Tetrafluorethylrest, einen 2,2,2-Trifluorethylrest oder einen Difluormethylrest darstellt; R1' Wasserstoff, Fluor, Methoxy oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind; n die Zahlen 0 oder 1 darstellt, und die Salze dieser Verbindungen.

8. Verbindungen der allgemeinen Formel I nach Anspruch 1, worin R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff, Fluor oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind; n die Zahlen 0 oder 1 darstellt, und die Salze dieser Verbindungen.

9. Verbindungen der allgemeinen Formel I nach Anspruch 1, in denen R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff bedeutet; R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

10. Verbindungen der allgemeinen Formel I nach Anspruch 1, in denen R1 einen Difluormethylrest darstellt, R1' Fluor, Methoxy oder Difluormethoxy bedeutet, R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

11. Verbindungen der allgemeinen Formel I nach Anspruch 1, worin R1 einen Trifluormethylrest, einen 1,1,2,2-Tetrafluorethylrest, einen 2,2,2-Trifluorethylrest oder einen Difluormethylrest darstellt; R1' Wasserstoff bedeutet; R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

12. Verbindungen der allgemeinen Formel I nach Anspruch 1, worin R1 einen Trifluormethylrest, einen 1,1,2,2-Tetrafluorethylrest, einen 2,2,2-Trifluorethylrest oder einen Difluormethylrest darstellt; R1' Fluor, Methoxy oder Difluormethoxy bedeutet; R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

13. Verbindungen der allgemeinen Formel I nach Anspruch 1, worin R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff bedeutet; R2 Wasserstoff oder Methyl, R3 Methoxy und R4 Wasserstoff oder Methyl bedeuten, n die Zahlen 0 oder 1 darstellt, und die pharmakologisch verträglichen Salze dieser Verbindungen.

**14.** Verbindungen nach einem der Ansprüche 1 bis 13 worin n die Zahl 0 bedeutet.

**15.** Verbindungen nach einem der Ansprüche 1 bis 13 worin n die Zahl 1 bedeutet.

**16.** Verbindung ausgewählt aus der Gruppe bestehend aus
2-[(4-Methoxy-2-pyridyl)methylsulfinyl]-5-trifluormethoxy-1H-benzimidazol,
2-[(4-Methoxy-3-methyl-2-pyridyl)methylthio]-5-trifluormethoxy-1H-benzimidazol,
2-[(4-Methoxy-3-methyl-2-pyridyl)methylsulfinyl]-5-trifluormethoxy-1H-benzimidazol,
2-[(4-Methoxy-5-methyl-2-pyridyl)methylsulfinyl]-5-trifluormethoxy-1H-benzimidazol,
2-[(4-Methoxy-2-pyridyl)methylsulfinyl]-5-(1,1,2,2-tetrafluorethoxy)-1H-benzimidazol,
2-[(4-Methoxy-3-methyl-2-pyridyl)methylsulfinyl]-5-(1,1,2,2-tetrafluorethoxy)-1H-benzimidazol,
2-[(4-Methoxy-3-methyl-2-pyridyl)methylthio]-5-(1,1,2,2-tetrafluorethoxy)-1H-benzimidazol,
2-[(4-Methoxy-3,5-dimethyl-2-pyridyl)methylsulfinyl]-5-trifluormethoxy-1H-benzimidazol,
2-[(4-Methoxy-3,5-dimethyl-2-pyridyl)methylthio]-5-trifluormethoxy-1H-benzimidazol,
2-[(4-Methoxy-3-methyl-2-pyridyl)methylsulfinyl]-5-(2,2,2-trifluorethoxy)-1H-benzimidazol,
2-[(4-Methoxy-3-methyl-2-pyridyl)methylthio]-5-(2,2,2-trifluorethoxy)-1H-benzimidazol,
5-Difluormethoxy-2-[(4-methoxy-3-methyl-2-pyridyl)-methylsulfinyl]-1H-benzimidazol,
5,6-Bis(difluormethoxy)-2-[(4-methoxy-2-pyridyl)methylsulfinyl]-1H-benzimidazol,
5,6-Bis(difluormethoxy)-2-[(4-methoxy-3-methyl-2-pyridyl]methylsulfinyl]-1H-benzimidazol,
5,6-Bis(difluormethoxy)-2-[(4-methoxy-3-methyl-2-pyridyl)methylthio]-1H-benzimidazol,
5-Difluormethoxy-6-methoxy-2-[(4-methoxy-3-methyl-2-pyridyl)methylsulfinyl]-1H-benzimidazol,
5-Difluormethoxy-6-methoxy-2-[(4-methoxy-3-methyl-2-pyridyl)methylthio]-1H-benzimidazol,
5-Chlordifluormethoxy-2-[(4-methoxy-2-pyridyl)methylsulfinyl]-1H-benzimidazol,
und ihren pharmakologisch verträglichen Salzen.

**17.** Verfahren zur Herstellung von Fluoralkoxyverbindungen der Formel I nach Anspruch 1 und ihren Salzen, dadurch gekennzeichnet, daß man

a) Mercaptobenzimidazole der Formel II mit Picolinderivaten III,

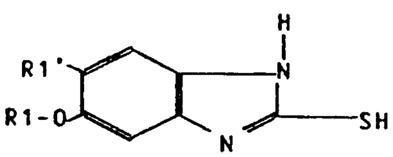 (II),

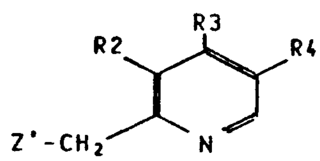 (III),

oder

b) Benzimidazole der Formel IV mit Mercaptopicolinen V,

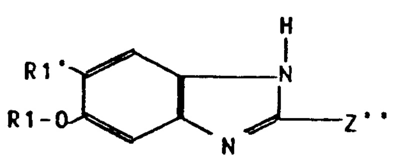 (IV),

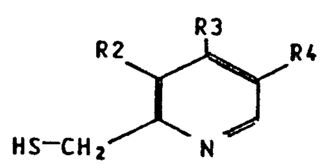 (V),

oder

c) o-Phenylendiamine der Formel VI mit Ameisensäurederivaten VII

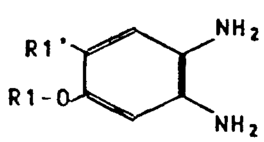 (VI),

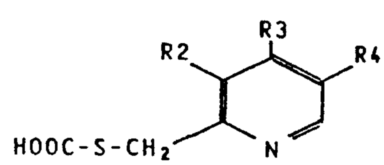 (VII),

umsetzt und gegebenenfalls anschließend die nach a), b) oder c) erhaltenen 2-Benzimidazolyl-2-pyridylmethyl-sulfide der Formel VIII

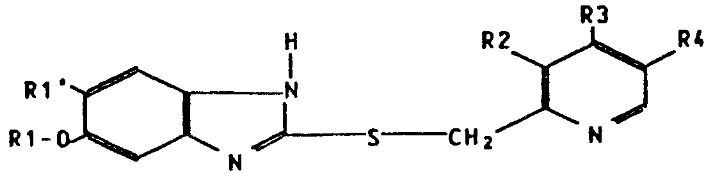 (VIII),

oxidiert und/oder in die Salze überführt,
oder daß man
d) Benzimidazole der Formel IX mit Pyridinderivaten X

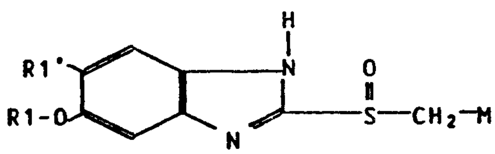 (IX), 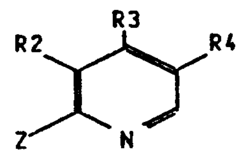 (X),

oder
e) Sulfinylderivate der Formel XI mit 2-Picolinderivaten XII

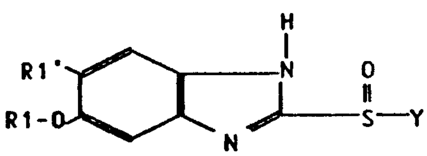 (XI), 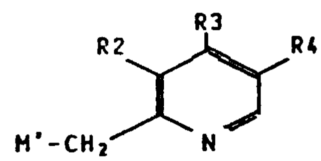 (XII),

umsetzt, und gegebenenfalls anschließend in die Salze überführt, wobei Y, Z, Z' und Z'' geeignete Abgangsgruppen darstellen, M für ein Alkalimetallatom (Li, Na oder K) steht, M' für das Äquivalent eines Metallatoms steht und R1, R1', R2, R3, R4 und n die in Anspruch 1 angegebenen Bedeutungen haben.

**18.** Fluoralkoxyverbindungen nach einem der Ansprüche 1 bis 16 und ihre pharmakologisch verträglichen Salze zur Anwendung bei der Behandlung beziehungsweise Prophylaxe von Krankheiten des Magens und/oder Darms und solcher Krankheiten, die auf einer erhöhten Magensäuresekretion beruhen.

**19.** Arzneimittel enthaltend ein oder mehrere Fluoralkoxyverbindungen nach einem oder mehreren der Ansprüche 1 bis 16 und/oder ihre pharmakologisch verträglichen Salze.

**20.** Mercaptobenzimidazole der Formel II

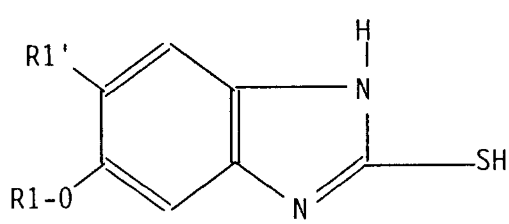 (II),

worin

R1 einen ganz oder überwiegend durch Fluor substituierten 1-3C-Alkylrest oder einen Chlordifluormethylrest und

R1' Wasserstoff, Halogen, Trifluormethyl, einen 1-3C-Alkylrest oder einen gegebenenfalls ganz oder überwiegend durch Fluor substituierten 1-3C-Alkoxyrest bedeutet,

sowie die Salze dieser Verbindungen.

**21.** Mercaptobenzimidazole der Formel II nach Anspruch 20,

worin

R1 einen Trifluormethylrest, einen 1,1,2,2-Tetrafluorethylrest, einen 2,2,2-Trifluorethylrest, einen Difluormethylrest oder einen Chlordifluormethylrest darstellt und

R1' Wasserstoff, Fluor, Methoxy oder Difluormethoxy bedeutet,

und die Salze dieser Verbindungen.

**Patentansprüche für folgenden Vertragsstaat : AT**

**1.** Verfahren zur Herstellung von Fluoralkoxyverbindungen der allgemeinen Formel I

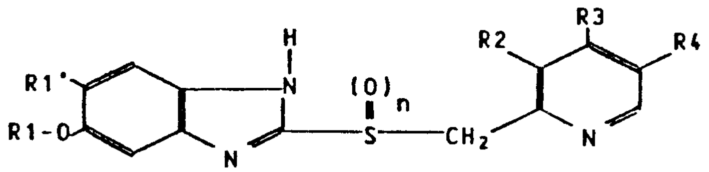

(I),

worin

R1 einen ganz oder überwiegend durch Fluor substituierten 1-3C-Alkylrest oder einen Chlordifluormethylrest,

R1' Wasserstoff, Halogen, Trifluormethyl, einen 1-3C-Alkylrest, oder einen gegebenenfalls ganz oder überwiegend durch Fluor substituierten 1-3C-Alkoxyrest,

R2 Wasserstoff oder einen 1-3C-Alkylrest,

R3 Wasserstoff, einen 1-3C-Alkyl- oder einen 1-3C-Alkoxyrest,

R4 Wasserstoff oder einen 1-3C-Alkylrest und

n die Zahlen 0 oder 1 darstellen,

und ihren Salzen, dadurch gekennzeichnet, daß man

a) Mercaptobenzimidazole der Formel II mit Picolinderivaten III,

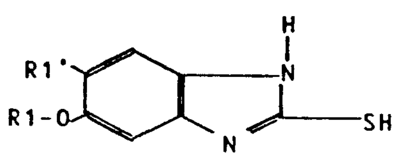

(II),

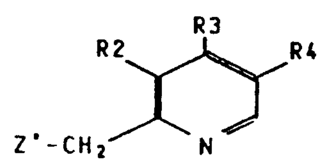

(III),

oder

b) Benzimidazole der Formel IV mit Mercaptopicolinen V,

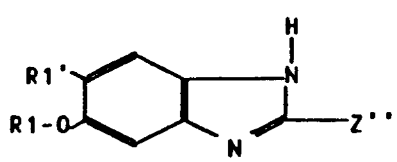

(IV),

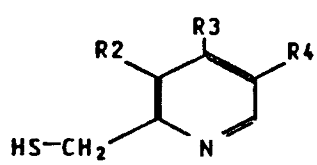

(V),

oder

c) o-Phenylendiamine der Formel VI mit Ameisensäurederivaten VII

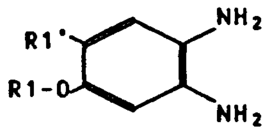 (VI), 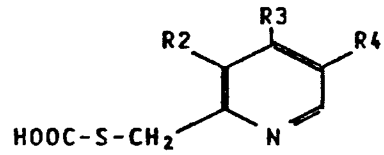 (VII),

umsetzt und gegebenenfalls anschließend die nach a), b) oder c) erhaltenen 2-Benzimidazolyl-2-pyridylmethyl-sulfide der Formel VIII

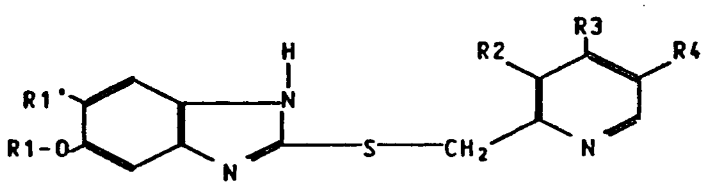 (VIII),

oxidiert und/oder in die Salze überführt,
oder daß man
d) Benzimidazole der Formel IX mit Pyridinderivaten X

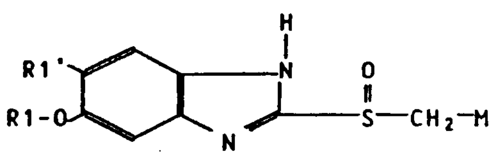 (IX), 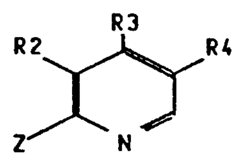 (X),

oder
e) Sulfinylderivate der Formel XI mit 2-Picolinderivaten XII

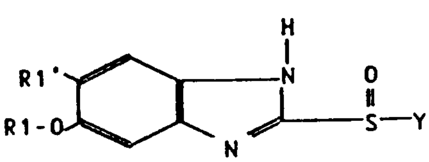 (XI), 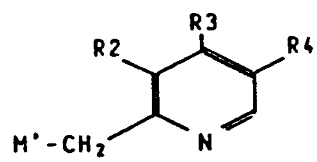 (XII),

umsetzt, und gegebenenfalls anschließend in die Salze überführt, wobei Y, Z, Z' und Z" geeignete Abgangsgruppen darstellen, M für ein Alkalimetallatom (Li, Na oder K) steht, M' für das Äquivalent eines Metallatoms steht und R1, R1', R2, R3, R4 und n die oben angegebenen Bedeutungen haben.

2. Verfahren nach Anspruch 1, worin R1 einen ganz oder überwiegend durch Fluor substituierten 1-3C-Alkylrest und R1' Wasserstoff, Halogen, Trifluormethyl oder einen ganz oder überwiegend durch Fluor substituierten 1-3C-Alkoxyrest darstellen; R2, R3, R4 und n die in Anspruch 1 angegebenen Bedeutungen haben.

3. Verfahren nach Anspruch 1, worin R1 einen ganz oder überwiegend durch Fluor substituierten 1-3C-Alkylrest darstellt und R1', R2, R3, R4 und n die in Anspruch 1 angegebenen Bedeutungen haben.

4. Verfahren nach Anspruch 1, worin R1' Wasserstoff, Halogen, Trifluormethyl oder einen ganz oder überwiegend durch Fluor substituierten 1-3C-Alkoxyrest darstellt und R1, R2, R3, R4 und n die in Anspruch 1 angegebenen Bedeutungen haben.

5. Verfahren nach Anspruch 1, worin R1 einen Trifluormethylrest, einen 1,1,2,2-Tetrafluorethylrest, einen 2,2,2-Trifluorethylrest, einen Difluormethylrest oder einen Chlordifluormethylrest darstellt: R1' Wasserstoff, Fluor, Methoxy oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind und n die Zahlen 0 oder 1 darstellt.

6. Verfahren nach Anspruch 1, worin R1 einen Trifluormethylrest, einen 1,1,2,2-Tetrafluorethylrest, einen 2,2,2-Trifluorethylrest oder einen Difluormethylrest darstellt; R1' Wasserstoff, Fluor oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind und n die Zahlen 0 oder 1 darstellt.

7. Verfahren nach Anspruch 1, worin R1 einen Trifluormethylrest, einen 1,1,2,2-Tetrafluorethylrest, einen 2,2,2-Trifluorethylrest oder einen Difluormethylrest darstellt; R1' Wasserstoff, Fluor, Methoxy oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind und n die Zahlen 0 oder 1 darstellt.

8. Verfahren nach Anspruch 1, worin R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff, Fluor oder Difluormethoxy bedeutet; R2 Wasserstoff oder Methyl, R3 Wasserstoff oder Methoxy und R4 Wasserstoff oder Methyl bedeuten, wobei R2, R3 und R4 nicht gleichzeitig Wasserstoffatome sind und n die Zahlen 0 oder 1 darstellt.

9. Verfahren nach Anspruch 1, worin R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff bedeutet; R2 Wasserstoff oder Methyl, R3 Methoxy und R4 Wasserstoff oder Methyl bedeuten und n die Zahlen 0 oder 1 darstellt.

10. Verfahren nach Anspruch 1, worin R1 einen Difluormethylrest darstellt, R1' Fluor, Methoxy oder Difluormethoxy bedeutet, R2 Wasserstoff oder Methyl, R3 Methoxy und R4 Wasserstoff oder Methyl bedeuten und n die Zahlen 0 oder 1 darstellt.

11. Verfahren nach Anspruch 1, worin R1 einen Trifluormethylrest, einen 1,1,2,2-Tetrafluorethylrest, einen 2,2,2-Trifluorethylrest oder einen Difluormethylrest darstellt; R1' Wasserstoff bedeutet; R2 Wasserstoff oder Methyl, R3 Methoxy und R4 Wasserstoff oder Methyl bedeuten und n die Zahlen 0 oder 1 darstellt.

12. Verfahren nach Anspruch 1, worin R1 einen Trifluormethylrest, einen 1,1,2,2-Tetrafluorethylrest, einen 2,2,2-Trifluorethylrest oder einen Difluormethylrest darstellt; R1' Fluor, Methoxy oder Difluormethoxy bedeutet; R2 Wasserstoff oder Methyl, R3 Methoxy und R4 Wasserstoff oder Methyl bedeuten und n die Zahlen 0 oder 1 darstellt.

13. Verfahren nach Anspruch 1, worin R1 einen Trifluormethylrest, einen 1,1,2,2-Tetrafluorethylrest, einen 2,2,2-Trifluorethylrest, einen Difluormethylrest oder einen Chlordifluormethylrest darstellt; R1' Wasserstoff bedeutet; R2 Wasserstoff oder Methyl, R3 Methoxy und R4 Wasserstoff oder Methyl bedeuten und n die Zahlen 0 oder 1 darstellt.

14. Verfahren nach Anspruch 1, worin n die Zahl 0 bedeutet, dadurch gekennzeichnet, daß man Mercaptobenzimidazole II mit Picolinderivaten III umsetzt.

15. Verfahren nach Anspruch 1 worin n die Zahl 1 bedeutet, dadurch gekennzeichnet, daß man 2-Benzimidazolyl-2-pyridylmethyl-sulfide VIII oxidiert.

16. Verfahren nach Anspruch 1, dadurch gekennzeichnet, daß
2-[(4-Methoxy-2-pyridyl)methylsulfinyl]-5-trifluormethoxy-1H-benzimidazol,
2-[(4-Methoxy-3-methyl-2-pyridyl)methylthio]-5-trifluormethoxy-1H-benzimidazol,
2-[(4-Methoxy-3-methyl-2-pyridyl)methylsulfinyl]-5-trifluormethoxy-1H-benzimidazol,

2-[(4-Methoxy-5-methyl-2-pyridyl)methylsulfinyl]-5-trifluormethoxy-1H-benzimidazol,
2-[(4-Methoxy-2-pyridyl)methylsulfinyl]-5-(1,1,2,2-tetrafluorethoxy)-1H-benzimidazol,
2-[(4-Methoxy-3-methyl-2-pyridyl)methylsulfinyl]-5-(1,1,2,2-tetrafluorethoxy)-1H-benzimidazol,
2-[(4-Methoxy-3-methyl-2-pyridyl)methylthio]-5-(1,1,2,2-tetrafluorethoxy)-1H-benzimidazol,
2-[(4-Methoxy-3,5-dimethyl-2-pyridyl)methylsulfinyl]-5-trifluormethoxy1H-benzimidazol,
2-[(4-Methoxy-3,5-dimethyl-2-pyridyl)methylthio]-5-trifluormethoxy-1H-benzimidazol,
2-[(4-Methoxy-3-methyl-2-pyridyl)methylsulfinyl)]-5-(2,2,2-trifluorethoxy)-1H-benzimidazol,
2-[(4-Methoxy-3-methyl-2-pyridyl)methylthio]-5-(2,2,2-trifluorethoxy)-1H-benzimidazol,
5-Difluormethoxy-2-[(4-methoxy-3-methyl-2-pyridyl)-methylsulfinyl]-1H-benzimidazol,
5,6-Bis(difluormethoxy)-2-[(4-methoxy-2-pyridyl)methylsulfinyl]-1H-benzimidazol,
5,6-Bis(difluormethoxy)-2-[(4-methoxy-3-methyl-2-pyridyl)methylsulfinyl]-1H-benzimidazol,
5,6-Bis(difluormethoxy)-2-[(4-methoxy-3-methyl-2-pyridyl)-methylthio]-1H-benzimidazol,
5-Difluormethoxy-6-methoxy-2-[(4-methoxy-3-methyl-2-pyridyl)methylsulfinyl]-1H-benzimidalol,
5-Difluormethoxy-6-methoxy-2-[(4-methoxy-3-methyl-2-pyridyl)methylthio]-1H-benzimidazol,
5-Chlordifluormethoxy-2-[(4-methoxy-2-pyridyl)methylsulfinyl]-1H-benzimidazol
oder ein pharmakologisch verträgliches Salz davon hergestellt wird.

**17.** Verfahren zur Herstellung Von Mercaptobenzimidazolen der Formel II,

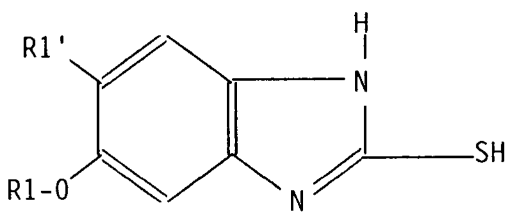

(II),

worin

R1 einen ganz oder überwiegend durch Fluor substituierten 1-3C-Alkylrest oder einen Chlordifluormethylrest und

R1' Wasserstoff, Halogen, Trifluormethyl, einen 1-3C-Alkylrest oder einen gegebenenfalls ganz oder überwiegend durch Fluor substituierten 1-3C-Alkoxyrest bedeutet, und ihren Salzen, dadurch gekennzeichnet, daß man

o-Phenylendiamine der Formel VI

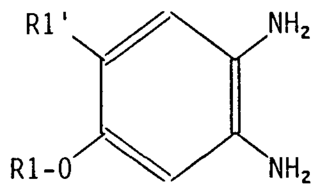

(VI),

mit Kohlendisulfid in Gegenwart von Alkalihydroxiden oder mit Alkali-0-ethyldithiocarbonaten umsetzt und gewünschtenfalls anschließend in die Salze überführt.

**18.** Verfahren nach Anspruch 17, wobei

R1 einen Trifluormethylrest, einen 1,1,2,2-Tetrafluorethylrest, einen 2,2,2-Trifluorethylrest, einen Difluormethylrest oder einen Chlordifluormethylrest darstellt und

R1' Wasserstoff, Fluor, Methoxy oder Difluormethoxy bedeutet.

## Claims

**Claims for the following Contracting States : BE, CH, DE, FR, GB, IT, LI, LU, NL, SE**

1. Fluoroalkoxy compounds of the general formula I,

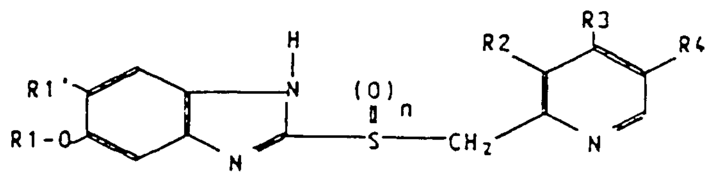

(I),

wherein

| | |
|---|---|
| R1 | represents a 1-3C-alkyl radical which is completely or predominantly substituted by fluorine, or a chlorodifluoromethyl radical, |
| R1' | represents hydrogen, halogen, trifluoromethyl, a 1-3C-alkyl radical, or a 1-3C-alkoxy radical which is optionally completely or predominantly substituted by fluorine, |
| R2 | represents hydrogen or a 1-3C-alkyl radical, |
| R3 | represents hydrogen or a 1-3C-alkyl or 1-3C-alkoxy radical, |
| R4 | represents hydrogen or a 1-3C-alkyl radical and |
| n | represents the number 0 or 1, |

and the salts of these compounds.

2. Compounds of the general formula I according to claim 1, wherein R1 represents a 1-3C-alkyl radical which is completely or predominantly substituted by fluorine, R1' represents hydrogen, halogen, trifluoromethyl or a 1-3C-alkoxy radical which is completely or predominantly substituted by fluorine, R2, R3, R4 and n have the meaning given in claim 1, and their salts.

3. Compounds of the general formula I according to claim 1, wherein R1 represents a 1-3C-alkyl radical which is completely or predominantly substituted by fluorine, and R1', R2, R3, R4 and n have the meaning given in claim 1, and their salts.

4. Compounds of the general formula I according to claim 1, wherein R1' represents hydrogen, halogen, trifluoromethyl, or a 1-3C-alkoxy radical which is completely or predominantly substituted by fluorine, and R1, R2, R3, R4 and n have the meaning given in claim 1, and their salts.

5. Compounds of the general formula I according to claim 1, wherein R1 represents a trifluoromethyl, 1,1,2,2-tetrafluoroethyl, 2,2,2-trifluoroethyl, difluoromethyl or chlorodifluoromethyl radical, R1' represents hydrogen, fluorine, methoxy or difluoromethoxy, R2 represents hydrogen or methyl, R3 represents hydrogen or methoxy, R4 represents hydrogen or methyl and wherein R2, R3, and R4 are not simultaneously hydrogen atoms, n represents the number 0 or 1, and the salts of these compounds.

6. Compounds of the general formula I according to claim 1, wherein R1 represents a trifluoromethyl, 1,1,2,2-tetrafluoroethyl, 2,2,2-trifluoroethyl, or difluoromethyl radical, R1' represents hydrogen, fluorine, or difluoromethoxy, R2 represents hydrogen or methyl, R3 represents hydrogen or methoxy, R4 represents hydrogen or methyl and wherein R2, R3 and R4 are not simultaneously hydrogen atoms, n represents the number 0 or 1, and the salts of these compounds.

7. Compounds of the general formula I according to claim 1, wherein R1 represents a trifluoromethyl, 1,1,2,2-tetrafluoroethyl, 2,2,2-trifluoroethyl, or difluoromethyl radical, R1' represents hydrogen, fluorine, methoxy or difluoromethoxy, R2 represents hydrogen or methyl, R3 represents hydrogen or methoxy, R4 represents hydrogen or methyl and wherein R2, R3 and R4 are not simultaneously hydrogen atoms, n represents the number 0 or 1, and the salts of these compounds.

8. Compounds of the general formula I according to claim 1, wherein R1 represents a trifluoromethyl, 1,1,2,2-tetrafluoroethyl, 2,2,2-trifluoroethyl, difluoromethyl, or chlorodifluoromethyl radical, R1' represents hydrogen, fluorine, or difluoromethoxy, R2 represents hydrogen or methyl, R3 represents hydrogen or methoxy, R4 represents hydrogen or methyl and wherein R2, R3 and R4 are not simultaneously hydrogen atoms, n represents the number 0 or 1, and the salts of these compounds.

**9.** Compounds of the general formula I according to claim 1, wherein R1 represents a trifluoromethyl, 1,1,2,2-tetrafluoroethyl, 2,2,2-trifluoroethyl, difluoromethyl or chlorodifluoromethyl radical, R1' represents hydrogen, R2 represents hydrogen or methyl, R3 represents methoxy, R4 represents hydrogen or methyl and n represents the number 0 or 1, and the pharmacologically acceptable salts of these compounds.

**10.** Compounds of the general formula I according to claim 1, wherein R1 represents a difluoromethyl radical, R1' represents fluorine, methoxy or difluoromethoxy, R2 represents hydrogen or methyl, R3 represents methoxy, R4 represents hydrogen or methyl and n represents the number 0 or 1, and the pharmacologically acceptable salts of these compounds.

**11.** Compounds of the general formula I according to claim 1, wherein R1 represents a trifluoromethyl, 1,1,2,2-tetrafluoroethyl, 2,2,2-trifluoroethyl or difluoromethyl radical, R1' represents hydrogen, R2 represents hydrogen or methyl, R3 represents methoxy, R4 represents hydrogen or methyl and n represents the number 0 or 1, and the parmacologically acceptable salts of these compounds.

**12.** Compounds of the general formula I according to claim 1, wherein R1 represents a trifluoromethyl, 1,1,2,2-tetrafluoroethyl, 2,2,2-trifluoroethyl or difluoromethyl radical, R1' represents fluorine, methoxy or difluoromethoxy, R2 repesents hydrogen or methyl, R3 represents methoxy, R4 represents hydrogen or methyl and n represents the number 0 or 1, and the pharmacologically acceptable salts of these compounds.

**13.** Compounds of the general formula I according to claim 1, wherein R1 represents a trifluoromethyl, 1,1,2,2-tetrafluoroethyl, 2,2,2-trifluoroethyl, difluoromethyl or chlorodifluoromethyl radical R1' represents hydrogen, R2 repesents hydrogen or methyl, R3 represents methoxy, R4 represents hydrogen or methyl and n represents the number 0 or 1, and the pharmacologically acceptable salts of these compounds.

**14.** Compounds according to one of the claims 1 to 13 wherein n represents the number 0.

**15.** Compounds according to one of the claims 1 to 13 wherein n represents the number 1.

**16.** A compound according to claim 1, selected from the group consisting of 2-[(4-methoxy-2-pyridyl)-methylsulfinyl]-5-trifluoromethoxy-1H-benzimidazole,
2-[(4-methoxy-3-methyl-2-pyridyl)methylthio]-5-trifluoromethoxy-1H-benzimidazole,
2-[(4-methoxy-3-methyl-2-pyridyl)methylsulfinyl]-5-trifluoromethoxy-1H-benzimidazole,
2-[(4-methoxy-5-methyl-2-pyridyl)methylsulfinyl]-5-trifluoromethoxy-1H-benzimidazole,
2-[(4-methoxy-2-pyridyl)methylsulfinyl]-5-(1,1,2,2-tetrafluoroethoxy)-1H-benzimidazole,
2-[(4-methoxy-3-methyl-2-pyridyl)methylsulfinyl]-5-(1,1,2,2-tetrafluoroethoxy)-1H-benzimidazole,
2[(4-methoxy-3-methyl-2-pyridyl)methylthio]-5-(1,1,2,2-tetrafluoroethoxy)-1H-benzimidazole,
2-[(4-methoxy-3,5-dimethyl-2-pyridyl)methylsulfinyl]-5-trifluoromethoxy-1H-benzimidazole,
2-[(4-methoxy-3,5-dimethyl-2-pyridyl)methylthio]-5-trifluoromethoxy-1H-benzimidazole,
2-[(4-methoxy-3-methyl-2-pyridyl)methylsulfinyl]-5-(2,2,2-trifluoroethoxy)-1H-benzimidazole,
2-[(4-methoxy-3-methyl-2-pyridyl)methylthio]-5-(2,2,2-trifluoroethoxy)-1H-benzimidazole,
5-difluoromethoxy-2-[(4-methoxy-3-methyl-2-pyridyl)methylsulfinyl]-1H-benzimidazole,
5,6-bis(difluoromethoxy)-2-[(4-methoxy-2-pyridyl)methylsulfinyl]-1H-benzimidazole,
5,6-bis(difluoromethoxy)-2-[(4-methoxy-3-methyl-2-pyridyl)methylsulfinyl]-1H-benzimidazole,
5,6-bis(difluoromethoxy)-2-[(4-methoxy-3-methyl-2-pyridyl)methylthio]-1H-benzimidazole,
5-difluoromethoxy-6-methoxy-2-[(4-methoxy-3-methyl-2-pyridyl)methylsulfinyl]-1H-benzimidazole,
5-difluoromethoxy-6-methoxy-2-[(4-methoxy-3-methyl-2-pyridyl)methylthio]-1H-benzimidazole,
5-chlorodifluoromethoxy-2-[(4-methoxy-2-pyridyl)methylsulfinyl]-1H-benzimidazole
and their pharmacologically compatible salts.

**17.** Process for the preparation of fluoroalkoxy compounds of the general formula I according to claim 1, and their salts, characterized in that

a) mercaptobenzimidazoles of the formula II are reacted with picoline derivatives III

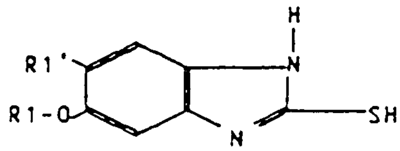 (II), 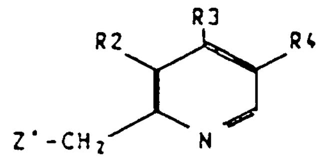 (III),

or

b) benzimidazoles of the formula IV are reacted with mercaptopicolines V

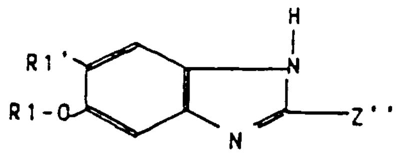 (IV), 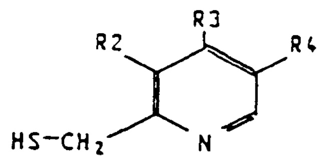 (V),

or

c) o-phenylenediamines of the formula VI are reacted with formic acid derivatives VII

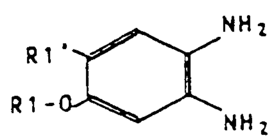 (VI), 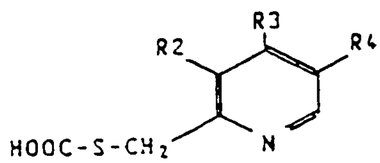 (VII),

and, if appropriate, the 2-benzimidazolyl 2-pyridyl sulfides obtained according to a), b) or c), of the formula VIII

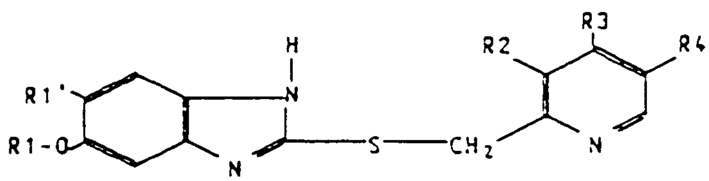 (VIII),

are then oxidized and/or converted into the salts,

or in that

d) benzimidazoles of the formula IX are reacted with pyridine derivatives X

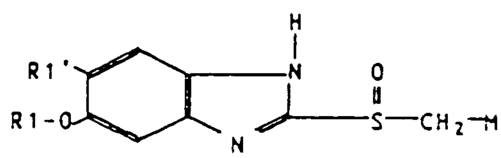 (IX), 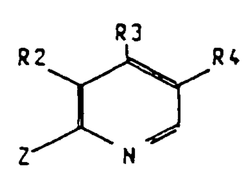 (X),

or

e) sulfinyl derivatives of the formula XI are reacted with 2-picoline derivatives XII

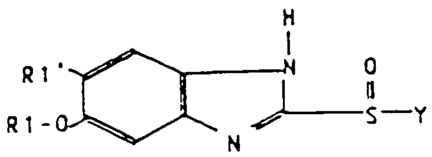 (XI), 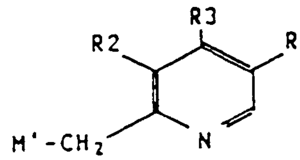 (XII),

and, if appropriate, the products are then converted into the salts, Y, Z, Z' and Z" representing suitable leaving groups, M representing an alkali metal atom (Li, Na or K), M' representing the equivalent of a metal atom and R1, R1', R2, R3, R4 and n having the meaning given in claim 1.

**18.** Fluoroalkoxy compounds according to any one of claims 1 to 16 and their pharmacologically acceptable salts for use in the treatment and prophylaxis of diseases of the stomach and/or intestine and/or those diseases based on an increased gastric acid secretion.

**19.** Medicaments containing one or more fluoroalkoxy compounds according to one or more of claims 1 to 16 and/or their pharmacologically acceptable salts.

**20.** Mercaptobenzimidazoles of formula II,

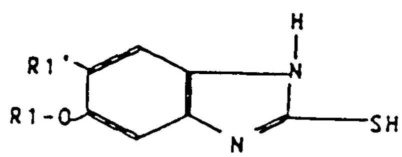 (II),

wherein
R1 represents a 1-3C-alkyl radical which is completely or predominantly substituted by fluorine, or a chlorodifluoromethyl radical and
R1' represents hydrogen, halogen, trifluoromethyl, a 1-3C-alkyl radical, or a 1-3C-alkoxy radical which his optionally completely or predominantly substituted by fluorine,
and the salts of these compounds.

**21.** Mercaptobenzimidazoles of formula II according to claim 20, wherein
R1 represents a trifluoromethyl, 1,1,2,2-tetrafluoroethyl, 2,2,2-trifluoroethyl, difluoromethyl or chlorodifluoromethyl radical and
R1' represents hydrogen, fluorine, methoxy or difluoromethoxy,
and the salts of these compounds.

**Claims for the following Contracting State : AT**

**1.** Process for the preparation of fluoroalkoxy compounds of the general formula I,

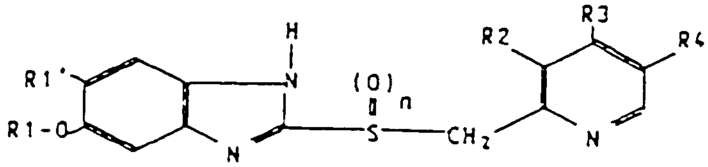 (I),

wherein
R1 represents a 1-3C-alkyl radical which is completely or predominantly substituted by fluorine, or a chlorodifluoromethyl radical,
R1' represents hydrogen, halogen, trifluoromethyl, a 1-3C-alkyl radical, or a 1-3C-alkoxy radical

which is optionally completely or predominantly substituted by fluorine,

R2 represents hydrogen or a 1-3C-alkyl radical,

R3 represents hydrogen or a 1-3C-alkyl or 1-3C-alkoxy radical,

R4 represents hydrogen or a 1-3C-alkyl radical and

n represents the number 0 or 1,

and their salts, characterized in that

a) mercaptobenzimidazoles of the formula II are reacted with picoline derivatives III

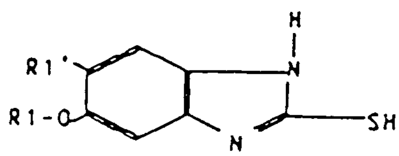 (II), 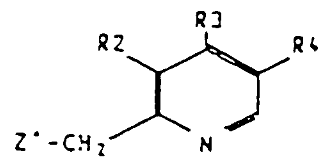 (III),

or

b) benzimidazoles of the formula IV are reacted with mercaptopicolines V

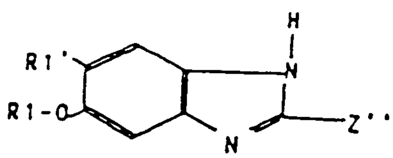 (IV), 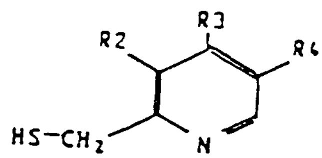 (V),

or

c) o-phenylenediamines of the formula VI are reacted with formic acid derivatives VII

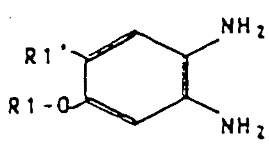 (VI), 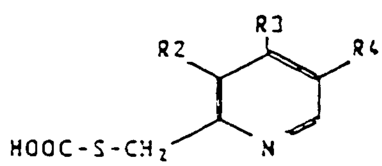 (VII),

and, if appropriate, the 2-benzimidazolyl 2-pyridyl sulfides obtained according to a), b) or c), of the formula VIII

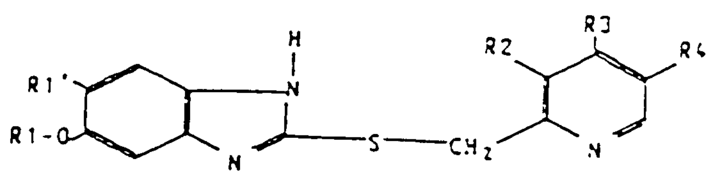 (VIII),

are then oxidized and/or converted into the salts,

or in that

d) benzimidazoles of the formula IX are reacted with pyridine derivatives X

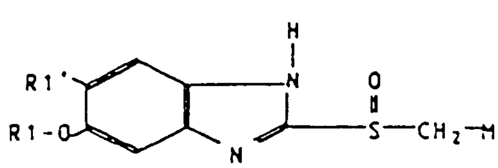 (IX), 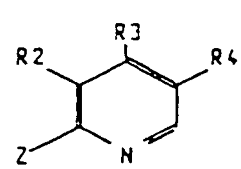 (X),

or

e) sulfinyl derivatives of the formula XI are reacted with 2-picoline derivatives XII

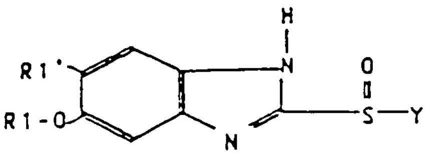

(XI),

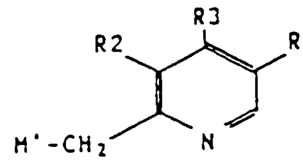

(XII),

and, if appropriate, the products are then converted into the salts, Y, Z, Z' and Z" representing suitable leaving groups, M representing an alkali metal atom (Li, Na or K), M' representing the equivalent of a metal atom and R1, R1', R2, R3, R4 and n having the above-mentioned meanings.

2. Process according to claim 1, wherein R1 represents a 1-3C-alkyl radical which is completely or predominantly substituted by fluorine, R1' represents hydrogen, halogen, trifluoromethyl or a 1-3C-alkoxy radical which is completely or predominantly substituted by fluorine, R2, R3, R4 and n have the meaning given in claim 1.

3. Process according to claim 1, wherein R1 represents a 1-3C-alkyl radical which is completely or predominantly substituted by fluorine, and R1', R2, R3, R4 and n have the meaning given in claim 1.

4. Process according to claim 1, wherein R1' represents hydrogen, halogen, trifluoromethyl, or a 1-3C-alkoxy radical which is completely or predominantly substituted by fluorine, and R1, R2, R3, R4 and n have the meaning given in claim 1.

5. Process according to claim 1, wherein R1 represents a trifluoromethyl, 1,1,2,2-tetrafluoroethyl, 2,2,2-trifluoroethyl, difluoromethyl or chlorodifluoromethyl radical, R1' represents hydrogen, fluorine, methoxy or difluoromethoxy, R2 represents hydrogen or methyl, R3 represents hydrogen or methoxy, R4 represents hydrogen or methyl and wherein R2, R3, and R4 are not simultaneously hydrogen atoms, n represents the number 0 or 1.

6. Process according to claim 1, wherein R1 represents a trifluoromethyl, 1,1,2,2-tetrafluoroethyl, 2,2,2-trifluoroethyl, or difluoromethyl radical, R1' represents hydrogen, fluorine, or difluoromethoxy, R2 represents hydrogen or methyl, R3 represents hydrogen or methoxy, R4 represents hydrogen or methyl and wherein R2, R3 and R4 are not simultaneously hydrogen atoms, n represents the number 0 or 1.

7. Process according to claim 1, wherein R1 represents a trifluoromethyl, 1,1,2,2-tetrafluoroethyl, 2,2,2-trifluoroethyl, or difluoromethyl radical, R1' represents hydrogen, fluorine, methoxy or difluoromethoxy, R2 represents hydrogen or methyl, R3 represents hydrogen or methoxy, R4 represents hydrogen or methyl and wherein R2, R3 and R4 are not simultaneously hydrogen atoms, n represents the number 0 or 1.

8. Process according to claim 1, wherein R1 represents a trifluoromethyl, 1,1,2,2-tetrafluoroethyl, 2,2,2-trifluoroethyl, difluoromethyl, or chlorodifluoromethyl radical, R1' represents hydrogen, fluorine, or difluoromethoxy, R2 represents hydrogen or methyl, R3 represents hydrogen or methoxy, R4 represents hydrogen or methyl and wherein R2, R3 and R4 are not simultaneously hydrogen atoms, n represents the number 0 or 1.

9. Process according to claim 1, wherein R1 represents a trifluoromethyl, 1,1,2,2-tetrafluoroethyl, 2,2,2-trifluoroethyl, difluoromethyl or chlorodifluoromethyl radical, R1' represents hydrogen, R2 represents hydrogen or methyl, R3 represents methoxy, R4 represents hydrogen or methyl and n represents the number 0 or 1.

10. Process according to claim 1, wherein R1 represents a difluoromethyl radical, R1' represents fluorine, methoxy or difluoromethoxy, R2 represents hydrogen or methyl, R3 represents methoxy, R4 represents hydrogen or methyl and n represents the number 0 or 1.

**11.** Process according to claim 1, wherein R1 represents a trifluoromethyl, 1,1,2,2-tetrafluoroethyl, 2,2,2-trifluoroethyl or difluoromethyl radical, R1' represents hydrogen, R2 represents hydrogen or methyl, R3 represents methoxy, R4 represents hydrogen or methyl and n represents the number 0 or 1.

**12.** Process according to claim 1, wherein R1 represents a trifluoromethyl, 1,1,2,2-tetrafluoroethyl, 2,2,2-trifluoroethyl or difluoromethyl radical, R1' represents fluorine, methoxy or difluoromethoxy, R2 repesents hydrogen or methyl, R3 represents methoxy, R4 represents hydrogen or methyl and n represents the number 0 or 1.

**13.** Process according to claim 1, wherein R1 represents a trifluoromethyl, 1,1,2,2-tetrafluoroethyl, 2,2,2-trifluoroethyl, difluoromethyl or chlorodifluoromethyl radical, R1' represents hydrogen, R2 repesents hydrogen or methyl, R3 represents methoxy, R4 represents hydrogen or methyl and n represents the number 0 or 1.

**14.** Process according to claim 1 wherein n represents the number 0, characterized in that mercaptobenzimidazoles II are reacted with picoline derivatives III.

**15.** Process according to claim 1 wherein n represents the number 1, characterized in that 2-benzimidazolyl 2-pyridyl sulfides VIII are oxidized.

**16.** Process according to claim 1, characterized in that
2-[(4-methoxy-2-pyridyl)methylsulfinyl]-5-trifluoromethoxy-1H-benzimidazole,
2-[(4-methoxy-3-methyl-2-pyridyl)methylthio]-5-trifluoromethoxy-1H-benzimidazole,
2-[(4-methoxy-3-methyl-2-pyridyl)methylsulfinyl]-5-trifluoromethoxy-1H-benzimidazole,
2-[(4-methoxy-5-methyl-2-pyridyl)methylsulfinyl]-5-trifluoromethoxy-1H-benzimidazole,
2-[(4-methoxy-2-pyridyl)methylsulfinyl]-5-(1,1,2,2-tetrafluoroethoxy)-1H-benzimidazole,
2-[(4-methoxy-3-methyl-2-pyridyl)methylsulfinyl]-5-(1,1,2,2-tetrafluoroethoxy)-1H-benzimidazole,
2-[(4-methoxy-3-methyl-2-pyridyl)methylthio]-5-(1,1,2,2-tetrafluoroethoxy)-1H-benzimidazole,
2-[(4-methoxy-3,5-dimethyl-2-pyridyl)methylsulfinyl]-5-trifluoromethoxy-1H-benzimidazole,
2-[(4-methoxy-3,5-dimethyl-2-pyridyl)methylthio]-5-trifluoromethoxy-1H-benzimidazole,
2-[(4-methoxy-3-methyl-2-pyridyl)methylsulfinyl]-5-(2,2,2-trifluoroethoxy)-1H-benzimidazole,
2-[(4-methoxy-3-methyl-2-pyridyl)methylthio]-5-(2,2,2-trifluoroethoxy)-1H-benzimidazole,
5-difluoromethoxy-2-[(4-methoxy-3-methyl-2-pyridyl)methylsulfinyl]-1H-benzimidazole,
5,6-bis(difluoromethoxy)-2-[(4-methoxy-2-pyridyl)methylsulfinyl]-1H-benzimidazole,
5,6-bis(difluoromethoxy)-2-[(4-methoxy-3-methyl-2-pyridyl)methylsulfinyl]-1H-benzimidazole,
5,6-bis(difluoromethoxy)-2-[(4-methoxy-3-methyl-2-pyridyl)methylthio]-1H-benzimidazole,
5-difluoromethoxy-6-methoxy-2-[(4-methoxy-3-methyl-2-pyridyl)methylsulfinyl]-1H-benzimidazole,
5-difluoromethoxy-6-methoxy-2-[(4-methoxy-3-methyl-2-pyridyl)methylthio]-1H-benzimidazole,
5-chlorodifluoromethoxy-2-[(4-methoxy-2-pyridyl)methylsulfinyl]-1H-benzimidazole
or a pharmacologically compatible salt thereof is prepared.

**17.** Process for the preparation of mercaptobenzimidazoles of formula II,

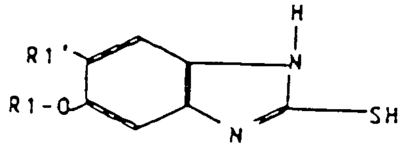

(II),

wherein

R1 represents a 1-3C-alkyl radical which is completely or predominantly substituted by fluorine, or a chlorodifluoromethyl radical and

R1' represents hydrogen, halogen, trifluoromethyl, a 1-3C-alkyl radical, or a 1-3C-alkoxy radical which his optionally completely or predominantly substituted by fluorine,

and their salts, characterized in that

o-phenylenediamines of formula VI

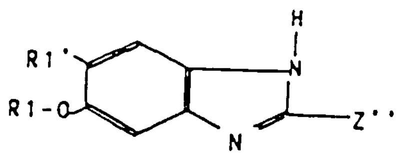

(IV).

are reacted with carbon disulfide in the presence of alkali metal hydroxides or with alkali metal o-ethyldithiocarbonates, and, if desired, are subsequently converted into the salts.

**18.** Process according to claim 17, whereby
R1 represents a trifluoromethyl, 1,1,2,2-tetrafluoroethyl, 2,2,2-trifluoroethyl, difluoromethyl or chlorodifluoromethyl radical and
R1' represents hydrogen, fluorine, methoxy or difluoromethoxy.

**Revendications**
**Revendications pour les Etats contractants suivants : BE, CH, DE, FR, GB, IT, LI, LU, NL, SE**

**1.** Composés fluoroalkoxy de formule générale I

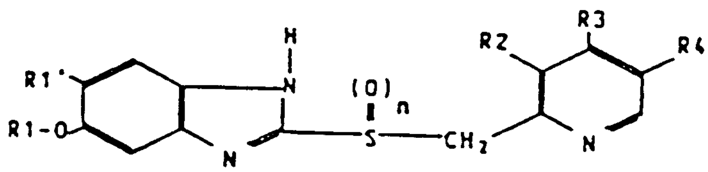

(I)

dans laquelle :
R1 représente un reste 1-3C-alkyle substitué totalement ou principalement par le fluor ou bien un reste chlorodifluorométhyle,
R1' représente l'hydrogène, un halogène, le trifluorométhyle, un reste 1-3C-alkyle, ou un reste 1-3C-alcoxy substitué le cas échéant totalement ou principalement par le fluor,
R2 représente l'hydrogène ou un reste 1-3C-alkyle,
R3 représente l'hydrogène, un groupe 1-3C-alkyle ou 1-3C-alcoxy,
R4 représente l'hydrogène ou un reste 1-3C-alkyle,
n représente les nombres 0 ou 1, ainsi que les sels de ces composés.

**2.** Composés de formule générale I selon la revendication 1, dans lesquels R1 représente un reste 1-3C-alkyle substitué totalement ou principalement par le fluor, et R1' l'hydrogène, les halogènes, le groupe trifluorométhyle ou un groupe 1-3C-alcoxy substitué totalement ou principalement par le fluor, R2, R3, R4 et n ont la signification indiquée dans la revendication 1, ainsi que les sels de ces composés.

**3.** Composés de formule générale I selon la revendication 1 dans lesquels R1 représente un reste 1-3C-alkyle substitué totalement ou principalement par le fluor et R1', R2, R3, R4 et n ont la signification indiquée dans la revendication 1, ainsi que les sels de ces composés.

**4.** Composés de formule générale I selon la revendication 1 dans lesquels R1' représente l'hydrogène, un halogène, le groupe trifluorométhyle, ou un groupe 1-3C-alcoxy substitué totalement ou principalement par le fluor et R1, R2, R3, R4 et n ont la signification indiquée dans la revendication 1, ainsi que les sels de ces composés.

**5.** Composés de formule générale I selon la revendication 1, dans lesquels R1 représente un reste trifluorométhyle, un reste 1,1,2,2-tétrafluoroéthyle, un reste 2,2,2-trifluoroéthyle, un reste difluorométhyle, ou bien un reste chlorodifluorométhyle, R1' représente l'hydrogène, le fluor, un groupe méthoxy ou difluorométhoxy, R2 représente l'hydrogène ou le groupe méthyle, R3 représente l'hydrogène ou un groupe méthoxy et R4 signifie l'hydrogène ou un groupe méthyle, R2, R3 et R4 ne sont pas simultanément des atomes d'hydrogène, n représente le nombre 0 ou 1, ainsi que les sels de ces composés.

6. Composés de formule générale I selon la revendication 1, dans lesquels R1 représente un groupe trifluorométhyle, un groupe 1,1,2,2-tétrafluoroéthyle, un groupe 2,2, 2-trifluoroéthyle, ou bien un groupe difluorométhyle ; R1' signifie l'hydrogène, le fluor ou le difluorométhoxy ; R2 représente l'hydrogène, un groupe méthyle, R3 l'hydrogène ou un groupe méthoxy, et R4 l'hydrogène ou un groupe méthyle, où R2, R3 et R4 ne sont pas simultanément l'hydrogène, n représente le nombre 0 ou 1, et les sels de ces composés.

7. Composés de formule générale I selon la revendication 1, dans lesquels R1 représente un groupe trifluorométhyle, un groupe 1,1,2,2-tétrafluoroéthyle, un groupe 2,2,2-trifluoroéthyle ou un groupe difluorométhyle, R1' signifie l'hydrogène, le fluor, le groupe méthoxy ou difluorométhoxy, R2 l'hydrogène ou le groupe méthyle, R3 l'hydrogène ou le groupe méthoxy, et R4 l'hydrogène ou un groupe méthyle, où R2, R3 et R4 ne sont pas simultanément l'hydrogène ; n représente le nombre 0 ou 1, ainsi que les sels de ces composés.

8. Composés de formule générale I selon la revendication 1, dans lesquels R1 représente un groupe trifluorométhyle, un groupe 1,1,2,2-tétrafluoroéthyle, un groupe 2,2, 2-trifluoroéthyle, un groupe difluorométhyle, ou un groupe chlorodifluorométhyle ; R1' représente l'hydrogène, le fluor ou un groupe difluorométhoxy, R2 représente l'hydrogène ou un groupe méthyle, R3 l'hydrogène ou un groupe méthoxy et R4 l'hydrogène ou un groupe méthyle où R2, R3 et R4 ne sont pas simultanément un atome d'hydrogène, n représente le nombre 0 ou 1, ainsi que les sels de ces composés.

9. Composés de formule générale I selon la revendication 1, dans lesquels R1 est un groupe trifluorométhyle, 1,1,2,2-tétrafluoroéthyle, 2,2,2-trifluoroéthyle, difluorométhyle ou chlorodifluorométhyle, R1' représente un atome d'hydrogène, R2, un atome d'hydrogène ou un groupe méthyle, R3, un groupe méthoxy et R4 un atome d'hydrogène ou un groupe méthyle, n représente les nombres 0 ou 1, ainsi que les sels pharmacologiquement acceptables de ces composés.

10. Composés de formule générale I selon la revendication 1, dans lesquels R1 représente un groupe difluorométhyle, R1', un atome de fluor, un groupe méthoxy ou difluorométhoxy, R2, un atome d'hydrogène, un groupe méthyle, R3, un groupe méthoxy et R4 un atome d'hydrogène ou un groupe méthyle, n représente les nombres 0 ou 1, ainsi que les sels pharmacologiquement acceptables de ces composés.

11. Composés de formule générale I selon la revendication 1, dans lesquels R1 représente un groupe trifluorométhyle, 1,1,2,2-tétrafluoroéthyle, 2,2,2-trifluoroéthyle ou difluorométhyle, R1', un atome d'hydrogène, R2, un atome d'hydrogène ou un groupe méthyle, R3, un groupe méthoxy et R4, un atome d'hydrogène ou un groupe méthyle, n représente les nombres 0 ou 1, ainsi que les sels pharmacologiquement acceptables de ces composés.

12. Composés de formule générale I selon la revendication 1, dans lesquels R1 représente un groupe trifluorométhyle, 1,1,2,2-tétrafluoroéthyle, 2,2,2-trifluoroéthyle ou difluorométhyle, R1' un atome de fluor, un groupe méthoxy ou difluorométhoxy, R2, un atome d'hydrogène ou un groupe méthyle, R3, un groupe méthoxy et R4, un atome d'hydrogène ou un groupe méthyle, n représente les nombres 0 ou 1, ainsi que les sels pharmacologiquement acceptables de ces composés.

13. Composés de formule générale I selon la revendication 1, dans lesquels R1 représente un groupe trifluorométhyle, 1,1,2,2-tétrafluoroéthyle, 2,2,2-trifluoroéthyle, difluorométhyle ou chlorodifluorométhyle, R1', un atome d'hydrogène, R2, un atome d'hydrogène ou un groupe méthyle, R3, un groupe méthoxy et R4, un atome d'hydrogène ou un groupe méthyle, n représente les nombres 0 ou 1, ainsi que les sels pharmacologiquement acceptables de ces composés.

14. Composés selon l'une des revendications 1 à 13, dans lesquels n représente le nombre 0.

15. Composés selon l'une des revendications 1 à 13, dans lesquels n représente le nombre 1.

16. Composés choisis dans le groupe constitué par :
2-[(4-méthoxy-2-pyridyl)méthylsulfinyl]-5-trifluorométhoxy-1H-benzimidazole.
2-[(4-méthoxy-3-méthyle-2-pyridyl)méthylthio]-5-trifluorométhoxy-1H-benzimidazole.

2-[(4-méthoxy-3-méthyl-2-pyridyl)méthylsulfinyl]5-trifluorométhoxy-1H-benzimidazole.
2-[(4-méthoxy-5-méthyl-2-pyridyl)méthylsulfinyl]-5-trifluorométhoxy-1H-benzimidazole.
2-[(4-méthoxy-2-pyridyl)méthylsulfinyl]-5-(1,1,2,2-tétrafluoroéthoxy)-1H-benzimidazole.
2-[(4-méthoxy-3-méthyl-2-pyridyl)méthylsulfinyl]-5-(1,1,2, 2-tétrafluoroéthoxy)-1H-benzimidazole.
2-[(4-méthoxy-3-méthyl-2-pyridyl)méthylthio]-5-(1,1,2,2-tétrafluoroéthoxy)-1H-benzimidazole.
2-[(4-méthoxy-3,5-diméthyl-2-pyridyl)méthylsulfinyl]-5-trifluorométhoxy-1H-benzimidazole.
2-[(4-méthoxy-3,5-diméthyl-2-pyridyl)méthylthio]-5-trifluorométhoxy-1H-benzimidazole.
2-[(4-méthoxy-3-méthyl-2-pyridyl)méthylsulfinyl]-5-(2,2,2-trifluoroéthory)-1H-benzimidazole.
2-[4-méthoxy-3-méthyl-2-pyridyl)méthylthio]-5-(2,2,2-trifluoroéthoxy)-1H-benzimidazole.
5-difluorométhoxy-2-[(4-méthoxy-3-méthyl-2-pyridyl)méthylsulfinyl]-1H-benzimidazole.
5,6-bis(difluorométhoxy)-2-[(4-méthoxy-2-pyridyl)méthylsulfinyl]-1H-benzimidazole.
5,6-bis(difluorométhoxy)-2-[(4-méthoxy-3-méthyl-2-pyridyl) méthylsulfiny]-1H-benzimidazole.
5,6-bis(difluorométhoxy)-2-[(4-méthoxy-3-méthyl-2-pyridyl) méthylthio]-1H-benzimidazole.
5-difluorométhoxy-6-méthoxy-2-[(4-méthoxy-3-méthyl-2-pyridyl) méthylsulfinyl]-1H-benzimidazole.
5-difluorométhoxy-6-méthoxy-2-[(4-méthoxy-3-méthyl-2-pyridyl) méthylthio]-1H-benzimidazole.
5-chlorodifluorométhoxy-2-[(4-méthoxy-2-pyridyl)méthylsulfinyl]-1H-benzimidazole ainsi que leurs sels pharmacologiquement acceptables.

**17.** Procédé de préparation des composés fluoroalcoxy de formule I selon la revendication 1 et de leurs sels, caractérisé en ce qu'on fait réagir :

a) un mercaptobenzimidazole de formule II avec un dérivé picoline de formule III,

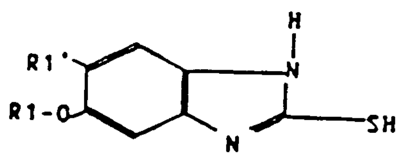

(II),

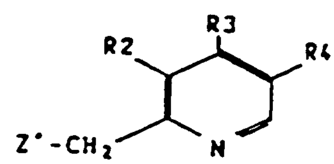

(III)

ou bien,

b) un benzimidazole de formule IV avec un mercaptopicoline de formule V :

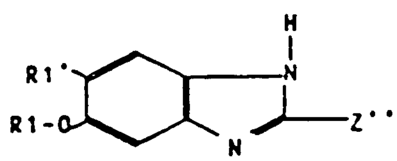

(IV),

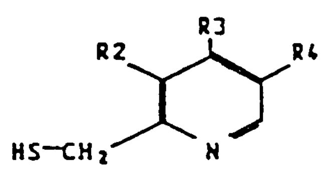

(V)

ou bien,

c) une o-phénylendiamine de formule VI avec un dérivé de l'acide formique de formule VII

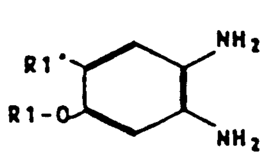

(VI),

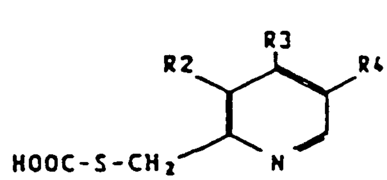

(VII)

et on oxyde le cas échéant, ensuite, le 2-benzimidazolyl-2-pyridylméthyl-sulfide de formule VIII

obtenu selon a), (b ou c).

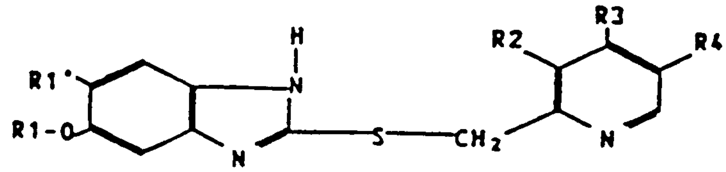 (VIII)

et/ou qu'on les transforme dans les sels correspondants, ou bien en ce qu'on fait réagir :
d) un benzimidazole de formule IX avec un dérivé pyridine de formule X

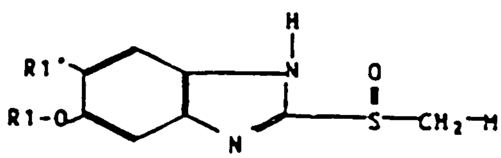 (IX), 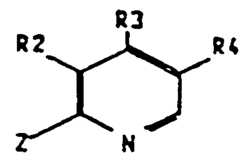 (X)

ou bien,
e) un dérivé sulfinyle de formule XI avec un dérivé 2-picoline de formule XII

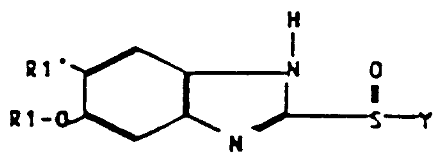 (XI), 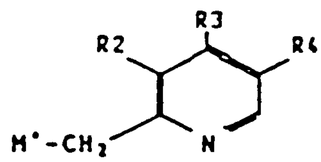 (XII)

et on forme ensuite, le cas échéant, les sels correspondants, où Y, Z, Z' et Z'' représentent des groupes labiles appropriés, M représente un métal alcalin (Li, Na ou K), M' représente l'équivalent d'un atome métallique, et R1, R1', R2, R3, R4 et n ont les significations indiquées dans la revendication 1.

**18.** Composés fluoroalcoxy selon l'une des revendications 1 à 16, ainsi que leurs sels pharmacologiquement acceptables utilisables pour le traitement ou la prolylaxie des maladies de l'estomac et/ou des intestins et des maladies qui résultent des sécrétions acides élevées de l'estomac.

**19.** Médicament renfermant un ou plusieurs composés fluoroalcoxy selon une ou plusieurs des revendications 1 à 16 et/ou leurs sels pharmacologiquement acceptables.

**20.** Mercaptobenzimidazole de formule II

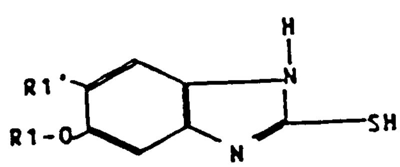 (II)

dans laquelle :
R1 représente un groupe alkyle 1-3C substitué totalement ou de manière prépondérante par le fluor ou un groupe chlorodifluorométhyle, et
R1' représente l'hydrogène, un halogène, le trifluorométhyle, un groupe 1-3C-alkyle ou un groupe 1-3C-alcoxy substitué, le cas échéant, totalement ou de manière prépondérante par le fluor, ainsi que les

sels de ces composés.

**21.** Mercaptobenzimidazole de formule II selon la revendication 20, dans lequel :
R1 représente un groupe trifluorométhyle, 1,1,2,2-tétrafluoroéthyle, 2,2,2-trifluoroéthyle, difluorométhyle ou chlorodifluorométhyle, et
R1' représente un atome d'hydrogène, de fluor, un groupe méthoxy ou difluorométhoxy, ainsi que les sels de ces composés.

**Revendications pour l'Etat contractant suivant : AT**

**1.** Procédé de préparation de composés fluoroalcoxy de formule générale I

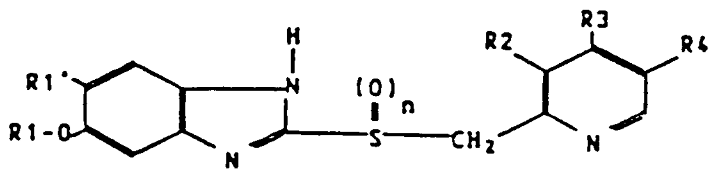

(I)

dans laquelle :
R1 représente un groupe 1-3C-alkyle substitué totalement ou de manière prépondérante par le fluor ou un groupe chlorodifluorométhyle,
R1', l'hydrogène ou un halogène, un groupe trifluorométhyle, 1-3C-alkyle, ou un groupe 1-3C-alcoxy substitué le cas échéant totalement ou de manière prépondérante par le fluor,
R2, l'hydrogène ou un groupe 1-3C-alkyle,
R3, l'hydrogène ou un groupe 1-3C-alkyle, ou 1-3C-alcoxy,
R4, l'hydrogène ou un groupe 1-3C-alkyle, et
n, le nombre 0 ou 1, ainsi que leurs sels, caractérisé en ce qu'on fait réagir :
a) un mercaptobenzimidazole de formule II avec un dérivé picoline de formule III,

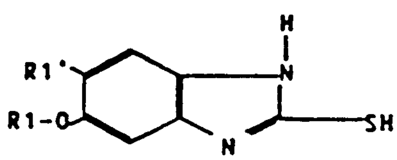

(II),

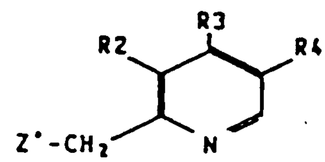

(III),

ou bien,
b) un benzimidazole de formule IV avec un mercaptopicoline de formule V :

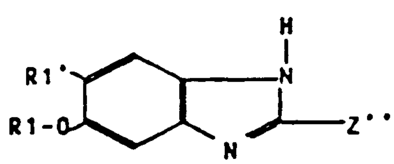

(IV),

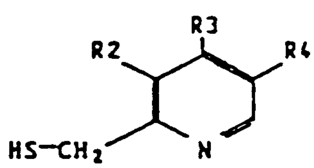

(V)

ou bien,
c) une o-phénylendiamine de formule VI avec un dérivé de l'acide formique de formule VII

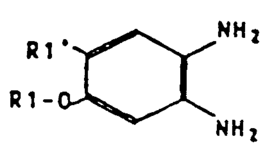

(VI),

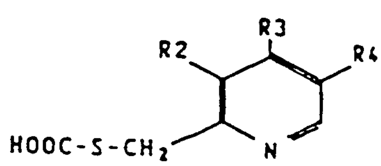

(VII).

et on oxyde, le cas échéant, ensuite, le 2-benzimidazolyl-2-pyridylméthyl-sulfide de formule VIII

obtenu selon a), b) ou c).

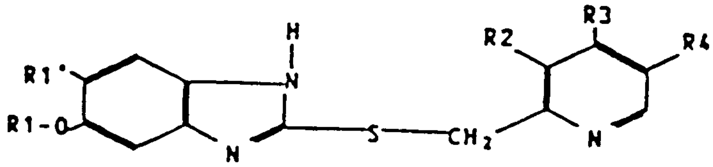 (VIII).

et/ou qu'on les transforme dans les sels correspondants, ou bien en ce qu'on fait réagir :
d) un benzimidazole de formule IX avec un dérivé pyridine de formule X

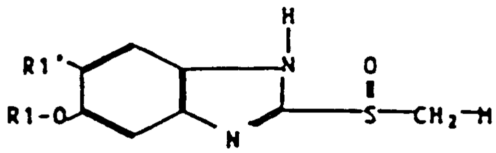 (IX), 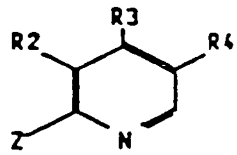 (X)

ou bien,
e) un dérivé sulfinyle de formule XI avec un dérivé 2-picoline de formule XII

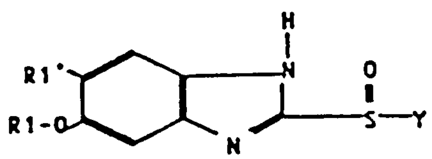 (XI), 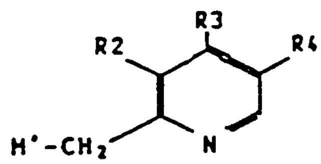 (XII)

et on forme ensuite, le cas échéant, les sels correspondants, où Y, Z, Z' et Z'' représentent des groupes labiles appropriés, M représente un métal alcalin (Li, Na ou K), M' représente l'équivalent d'un atome métallique, et R1, R1', R2, R3, R4 et n ont les significations indiquées ci-dessus.

**2.** Procédé selon la revendication 1, caractérisé en ce que R1 représente un reste 1-3C-alkyle substitué totalement ou principalement par le fluor, et R1' l'hydrogène, les halogènes, le groupe trifluorométhyle ou un groupe 1-3C-alcoxy substitué totalement ou principalement par le fluor, R2, R3, R4 et n ont la signification indiquée dans la revendication 1.

**3.** Procédé selon la revendication 1, dans lequel R1 représente un groupe 1-3C-alkyle substitué totalement ou principalement par le fluor, et R1', R2, R3, R4 et n ont la signification indiquée dans la revendication 1.

**4.** Procédé selon la revendication 1, dans lequel R1' représente l'hydrogène, un halogène, le groupe trifluorométhyle ou un groupe 1-3C-alcoxy substitué totalement ou de manière prépondérante par le fluor et R1, R2, R3, R4 et n ont la signification indiquée dans la revendication 1.

**5.** Procédé selon la revendication 1, dans lequel R1 représente un reste trifluorométhyle, un reste 1,1,2,2-tétrafluoroéthyle, un reste 2,2,2-trifluoroéthyle, un reste difluorométhyle, ou bien un reste chlorodifluorométhyle, R1' représente l'hydrogène, le fluor, un groupe méthoxy ou difluorométhoxy, R2 représente l'hydrogène ou le groupe méthyle, R3 représente l'hydrogène ou un groupe méthoxy et R4 signifie l'hydrogène ou un groupe méthyle, R2, R3 et R4 ne sont pas simultanément des atomes d'hydrogène, n représente le nombre 0 ou 1.

**6.** Procédé selon la revendication 1, dans lequel R1 représente un groupe trifluorométhyle, un groupe 1,1,2,2-tétrafluoroéthyle, un groupe 2,2,2-trifluoroéthyle, ou bien un groupe difluorométhyle ; R1' signifie l'hydrogène, le fluor ou le difluorométhoxy ; R2 représente l'hydrogène, un groupe méthyle, R3, l'hydrogène ou un groupe méthoxy, et R4 l' hydrogène ou un groupe méthyle, où R2, R3 et R4 ne sont pas simultanément l'hydrogène, n représente le nombre 0 ou 1.

7. Procédé selon la revendication 1, dans lequel R1 représente un groupe trifluorométhyle, un groupe 1,1,2, 2-tétrafluoroéthyle, un groupe 2,2,2-trifluoroéthyle ou un groupe difluorométhyle, R1' signifie l'hydrogène, le fluor, le groupe méthoxy ou difluorométhoxy, R2, l'hydrogène ou le groupe méthyle, R3, l'hydrogène ou le groupe méthoxy, et R4 l'hydrogène ou un groupe méthyle, où R2, R3 et R4 ne sont pas simultanément l'hydrogène ; n représente le nombre 0 ou 1.

8. Procédé selon la revendication 1, dans lequel R1 représente un groupe trifluorométhyle, un groupe 1,1,2,2-tétrafluoroéthyle, un groupe 2,2,2-trifluoroéthyle, un groupe difluorométhyle ou un groupe chlorodifluorométhyle ; R1' représente l'hydrogène, le fluor ou un groupe difluorométhoxy, R2 représente l'hydrogène ou un groupe méthyle, R3, l'hydrogène ou un groupe méthoxy et R4, l'hydrogène ou un groupe méthyle où R2, R3 et R4 ne sont pas simultanément un atome d'hydrogène, n représente le nombre 0 ou 1.

9. Procédé selon la revendication 1, dans lequel R1 est un groupe trifluorométhyle, 1,1,2,2-tétrafluoroéthyle, 2,2,2-trifluoroéthyle, difluorométhyle ou chlorodifluorométhyle, R1' représente un atome d'hydrogène, R2, un atome d'hydrogène ou un groupe méthyle, R3, un groupe méthoxy et R4, un atome d'hydrogène ou un groupe méthyle, n représente les nombres 0 ou 1.

10. Procédé selon la revendication 1, dans lequel R1 représente un groupe difluorométhyle, R1' signifie le fluor, un groupe méthoxy ou difluorométhoxy, R2, l'hydrogène ou un groupe méthyle, R3, un groupe méthoxy, R4, l'hydrogène ou un groupe méthyle, et n représente le nombre 0 ou 1.

11. Procédé selon la revendication 1, dans lequel R1 représente un groupe trifluorométhyle, 1,1,2,2-tétrafluoroéthyle, 2,2,2-trifluoroéthyle ou difluorométhyle, R1' un atome d'hydrogène, R2, un atome d'hydrogène ou un groupe méthyle, R3, un groupe méthoxy et R4, un atome d'hydrogène ou un groupe méthyle, n représente les nombres 0 ou 1.

12. Procédé selon la revendication 1, dans lequel R1 représente un groupe trifluorométhyle, 1,1,2,2-tétrafluoroéthyle, 2,2,2-trifluoroéthyle ou difluorométhyle, R1', un atome de fluor, un groupe méthoxy ou difluorométhoxy, R2, un atome d'hydrogène ou un groupe méthyle, R3, un groupe méthoxy et R4, un atome d'hydrogène ou un groupe méthyle, n représente les nombres 0 ou 1.

13. Procédé selon la revendication 1, dans lequel R1 représente un groupe trifluorométhyle, 1,1,2,2-tétrafluoroéthyle, 2,2,2-trifluoroéthyle, difluorométhyle ou chlorodifluorométhyle, R1', un atome d'hydrogène, R2, un atome d'hydrogène ou un groupe méthyle, R3, un groupe méthoxy et R4, un atome d'hydrogène ou un groupe méthyle, n représente les nombres 0 ou 1.

14. Procédé selon la revendication 1, dans lequel n représente le nombre 0, caractérisé en ce qu'on fait réagir le mercaptobenzimidazole de formule II avec le dérivé picoline de formule III.

15. Procédé selon la revendication 1, dans lequel n représente le nombre 1, caractérisé en ce qu'on oxyde le 2-benzimidazolyl-2-pyridylméthyl-sulfide de formule VIII.

16. Procédé selon la revendication 1, caractérisé en ce qu'on prépare :
2-[(4-méthoxy-2-pyridyl)méthylsulfinyl]-5-trifluorométhoxy-1H-benzimidazole.
2-[(4-méthoxy-3-méthyle-2-pyridyl)méthylthio]-5-trifluorométhoxy-1H-benzimidazole.
2-[(4-méthoxy-3-méthyl-2-pyridyl)méthylsulfinyl]-5-trifluorométhoxy-1H-benzimidazole.
2-[(4-méthoxy-5-méthyl-2-pyridyl)méthylsulfinyl]-5-trifluorométhoxy-1H-benzimidazole.
2-[(4-méthoxy-2-pyridyl)méthylsulfinyl]-5-(1,1,2,2-tétrafluoroéthoxy)-1H-benzimidazole.
2-[(4-méthoxy-3-méthyl-2-pyridyl)méthylsulfinyl]-5-(1,1,2, 2-tétrafluoroéthoxy)-1H-benzimidazole.
2-[(4-méthoxy-3-méthyl-2-pyridyl)méthylthio]-5-(1,1,2,2-tétrafluoroéthoxy)-1H-benzimidazole.
2-[(4-méthoxy-3,5-diméthyl-2-pyridyl)méthylsulfinyl]-5-trifluorométhoxy-1H-benzimidazole.
2-[(4-méthoxy-3,5-diméthyl-2-pyridyl)méthylthio]-5-trifluorométhoxy-1H-benzimidazole.
2-[(4-méthoxy-3-méthyl-2-pyridyl)méthylsulfinyl]-5-(2,2,2-trifluoroéthoxy)-1H-benzimidazole.
2-[(4-méthoxy-3-méthyl-2-pyridyl)méthylthio]-5-(2,2,2-trifluoroéthoxy)-1H-benzimidazole.
5-difluorométhoxy-2-[(4-méthoxy-3-méthyl-2-pyridyl)méthylsulfinyl]-1H-benzimidazole.
5,6-bis(difluorométhoxy)-2-[(4-méthoxy-2-pyridyl)méthylsulfinyl]-1H-benzimidazole.
5,6-bis(difluorométhoxy)-2-[(4-méthoxy-3-méthyl-2-pyridyl) méthylsulfinyl]-1H-benzimidazole.

5,6-bis(difluorométhoxy)-2-[(4-méthoxy-3-méthyl-2-pyridyl) méthylthio]-1H-benzimidazole.
5-difluorométhoxy-6-méthoxy-2-[(4-méthoxy-3-méthyl-2-pyridyl) méthylsulfinyl]-1H-benzimidazole.
5-difluorométhoxy-6-méthoxy-2-[(4-méthoxy-3-méthyl-2-pyridyl) méthylthio]-1H-benzimidazole.
5-chlorodifluorométhoxy-2-[(4-méthoxy-2-pyridyl)méthylsulfinyl]-1H-benzimidazole ainsi que leurs sels pharmacologiquement acceptables.

**17.** Procédé de préparation d'un mercaptobenzimidazole de formule II

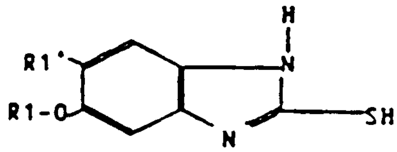

(II).

dans laquelle :
R1 représente un groupe 1-3C-alkyle substitué totalement ou de manière prépondérante par le fluor ou un groupe chlorodifluorométhyle, et
R1' représente l'hydrogène, un halogène, un groupe trifluorométhyle, un groupe 1-3C-alkyle ou un groupe 1-3C-alcoxy substitué, le cas échéant, totalement ou de manière prépondérante, par le fluor, et de leurs sels, caractérisé en ce qu'on fait réagir une o-phénylendiamine de formule VI

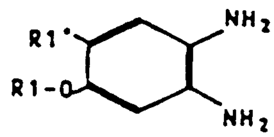

(VI).

avec le disulfide de carbone en présence d'un hydroxyde alcalin ou d'un alcalin-o-éthyldithiocarbonate et on transforme, si besoin est, ensuite, en sel.

**18.** Procédé selon la revendication 17 où : R1 représente un groupe trifluorométhyle, 1,1,2,2-tétrafluoroéthyle, 2,2,2-trifluoroéthyle, difluorométhyle ou chlorodifluorométhyle, et
R1' représente un atome d'hydrogène, de fluor, un groupe méthoxy ou difluorométhoxy.